(19) Europäisches Patentamt
European Patent Office
Office européen des brevets

(11) EP 4 241 770 A1

(12) EUROPEAN PATENT APPLICATION
published in accordance with Art. 153(4) EPC

(43) Date of publication:
**13.09.2023 Bulletin 2023/37**

(21) Application number: **21888642.2**

(22) Date of filing: **05.11.2021**

(51) International Patent Classification (IPC):
***A61K 31/407*** (2006.01) ***A61K 31/437*** (2006.01)
***A61K 31/522*** (2006.01) ***A61K 31/7072*** (2006.01)
***A61K 45/06*** (2006.01) ***A61P 31/20*** (2006.01)

(52) Cooperative Patent Classification (CPC):
**A61K 31/407; A61K 31/437; A61K 31/522; A61K 31/7072; A61K 45/06; A61P 31/20**

(86) International application number:
**PCT/CN2021/128892**

(87) International publication number:
**WO 2022/095950 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.11.2020 CN 202011222818**
**22.07.2021 CN 202110830556**

(71) Applicant: **CHIA TAI TIANQING PHARMACEUTICAL GROUP CO., LTD.**
**Lianyungang,**
**Jiangsu 222062 (CN)**

(72) Inventors:
- **XU, Zhongnan**
  **Lianyungang, Jiangsu 222062 (CN)**
- **HUO, Dandan**
  **Lianyungang, Jiangsu 222062 (CN)**
- **ZHANG, Hong**
  **Lianyungang, Jiangsu 222062 (CN)**
- **YU, Yanan**
  **Lianyungang, Jiangsu 222062 (CN)**
- **XIA, Jianhua**
  **Lianyungang, Jiangsu 222062 (CN)**
- **HE, Haiying**
  **Lianyungang, Jiangsu 222062 (CN)**

(74) Representative: **Grünecker Patent- und Rechtsanwälte**
**PartG mbB**
**Leopoldstraße 4**
**80802 München (DE)**

# (54) PHARMACEUTICAL COMBINATION CONTAINING CAPSID PROTEIN INHIBITOR AND NUCLEOSIDE ANALOG

(57) A pharmaceutical combination containing a capsid protein inhibitor and a nucleoside analog. Specifically, the present invention relates to a pharmaceutical combination of a capsid protein inhibitor and a nucleoside analog selected from entecavir or a pharmaceutically acceptable salt thereof or a solvate thereof, or tenofovir or a pharmaceutically acceptable salt or pharmaceutically acceptable prodrug thereof, as well as a use of said pharmaceutical combination in treating a hepatitis B virus infection. The present pharmaceutical combination has a good hepatitis B virus infection-fighting effect.

EP 4 241 770 A1

## Description

### TECHNICAL FIELD

The present application is in the field of medicinal chemistry and relates to a pharmaceutical combination comprising a capsid protein inhibitor and a nucleoside analog. In particular, the present application relates to a pharmaceutical combination of a compound of formula I as a capsid protein inhibitor and entecavir or tenofovir, or use thereof in treating hepatitis B virus infection.

### BACKGROUND

According to World Health Organization statistics, about 257 million people worldwide are infected with the hepatitis B virus (HBV). If not well treated, patients with hepatitis will be confronted with long-term fatal diseases such as liver failure, cirrhosis and liver cancer.

In patients infected with HBV, stable covalently closed circular DNA (cccDNA) is formed in the nuclei of hepatocytes of the host as a template for HBV replication. Subgenomic RNAs (sgRNAs) and pregenomic RNAs (pgRNAs) are all formed by cccDNA transcription. After exiting the nuclei, sgRNAs are translated into X proteins and three other envelope proteins, and pgRNAs into core proteins and viral polymerases. pgRNAs and core proteins self-assemble under the action of polymerases to form nucleocapsid-enveloped RNAs. Within nucleocapsids, pgRNAs are reverse-transcribed into negative DNA strands and positive DNA strands are further synthesized according to them, so that rcDNAs are formed. Nucleocapsid-enveloped rcDNAs are, in one aspect, re-shelled and enter the nuclei to further amplify cccDNA and, in another aspect, re-bind to envelope protein and are released from cells through the endoplasmic reticulum to form new HBV In the replication cycles of HBV, the synthesis of nucleocapsid is a key step in the replication process of the HBV genome. The synthesis of the viral DNA can only occur specifically within nucleocapsids. The assembly of nucleocapsids is a revolution-restricting process that limits the diversity of HBV and is very sensitive to even subtle molecular interference. For the development of new therapies targeting various genotypes and drug-resistant strains of the hepatitis B virus, the targets acting on the synthesis and degradation of nucleocapsids are very promising.

Common therapies currently approved for the treatment of chronic hepatitis B include nucleoside (nucleotide) compounds and interferons. Nucleoside (nucleotide) drugs such as entecavir and tenofovir can inhibit the replication of the DNA of HBV

Although patients infected with the hepatitis B virus have many treatment options, there is still a need for more effective therapeutic agents for clinical use.

### SUMMARY

In one aspect, the present application provides a pharmaceutical combination comprising a compound of formula **I** or a pharmaceutically acceptable salt thereof, and a reverse transcriptase inhibitor or a pharmaceutically acceptable prodrug thereof or a pharmaceutically acceptable salt thererof or a solvate thereof,

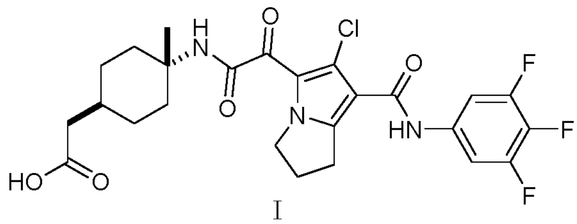

I

In some embodiments, the reverse transcriptase inhibitor is selected from the group consisting of entecavir or a pharmaceutically acceptable salt thereof or a solvate thereof, and tenofovir or a pharmaceutically acceptable prodrug thereof or a pharmaceutically acceptable salt thereof.

In one aspect, the present application provides a pharmaceutical combination comprising a compound of formula **I** or a pharmaceutically acceptable salt thereof, and entecavir or a pharmaceutically acceptable salt thereof or a solvate thereof.

In yet another aspect, the present application provides a pharmaceutical combination comprising a compound of formula **I** or a pharmaceutically acceptable salt thereof, and tenofovir or a pharmaceutically acceptable prodrug thereof or a pharmaceutically acceptable salt thereof.

In another aspect, the present application further provides use of the pharmaceutical combination of the present

application for preparing a medicament for treating hepatitis B virus infection. The present application further provides a method for treating hepatitis B virus infection comprising administering to an individual in need an effective amount of the pharmaceutical combination of the present application. The present application further provides the pharmaceutical combination for use in treating hepatitis B virus infection. The present application further provides use of the pharmaceutical combination of the present application for treating hepatitis B virus infection.

In yet another aspect, the present application provides a pharmaceutical combination comprising a compound of formula **I** as shown below or a pharmaceutically acceptable salt thereof, and entecavir or a pharmaceutically acceptable salt thereof or a solvate thereof:

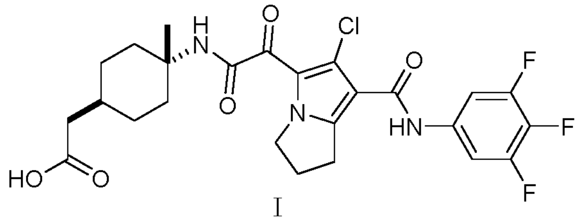

I

In some embodiments of the present application, the entecavir solvate is selected from an entecavir hydrate. In some embodiments, the entecavir hydrate is selected from hemihydrate to dihydrate of entecavir. In some embodiments, the entecavir hydrate is selected from entecavir monohydrate.

In some embodiments of the present application, the pharmaceutically acceptable salt of entecavir is selected from a maleate. In some embodiments of the present application, the pharmaceutically acceptable salt of entecavir is selected from a monomaleate.

In some embodiments of the present application, entecavir or the pharmaceutically acceptable salt thereof or the solvate thereof is selected from the group consisting of entecavir monomaleate, entecavir monohydrate, and entecavir monomaleate monohydrate.

In some embodiments of the present application, the pharmaceutical combination described herein comprises a compound of formula **I** or a pharmaceutically acceptable salt thereof, and entecavir or a pharmaceutically acceptable salt thereof or a hydrate thereof. In some embodiments, the pharmaceutical combination described herein comprises a compound of formula **I** or a pharmaceutically acceptable salt thereof, and entecavir or entecavir monohydrate. In some embodiments, the pharmaceutical combination described herein comprises a compound of formula **I** or a pharmaceutically acceptable salt thereof, and entecavir monomaleate monohydrate.

In some embodiments of the present application, the pharmaceutically acceptable prodrug of tenofovir includes tenofovir disoproxil, tenofovir alafenamide, or tenofovir amibufenamide.

In some embodiments of the present application, the pharmaceutically acceptable salt of tenofovir or the pharmaceutically acceptable prodrug thereof is selected from the group consisting of a fumarate, an orotate, a disulfonate, a phosphate, a succinate, and an aspartate. In some embodiments of the present application, the pharmaceutically acceptable salt of tenofovir or the pharmaceutically acceptable prodrug thereof is selected from a fumarate.

In some embodiments of the present application, tenofovir or the pharmaceutically acceptable prodrug thereof or the pharmaceutically acceptable salt thereof is selected from the group consisting of tenofovir, tenofovir alafenamide fumarate, tenofovir disoproxil fumarate, tenofovir disoproxil orotate, tenofovir disoproxil hemidisulfonate, tenofovir disoproxil phosphate, tenofovir disoproxil succinate, tenofovir disoproxil aspartate, and tenofovir amibufenamide fumarate. In some embodiments of the present application, tenofovir or the pharmaceutically acceptable prodrug thereof or the pharmaceutically acceptable salt thereof is selected from the group consisting of tenofovir disoproxil fumarate and tenofovir alafenamide fumarate. In some embodiments of the present application, tenofovir or the pharmaceutically acceptable prodrug thereof or the pharmaceutically acceptable salt thereof is selected from tenofovir disoproxil fumarate.

In some embodiments of the present application, tenofovir or the pharmaceutically acceptable prodrug thereof or the pharmaceutically acceptable salt thereof is selected from the group consisting of the following structures:

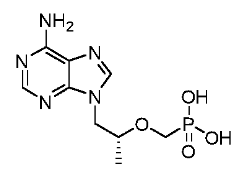

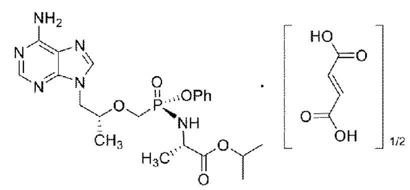

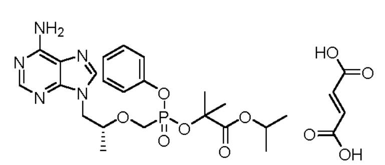

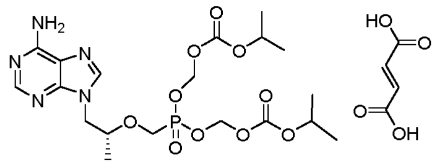

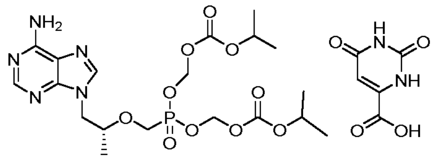

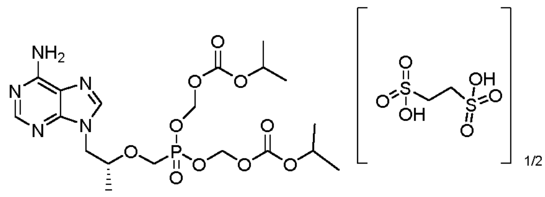

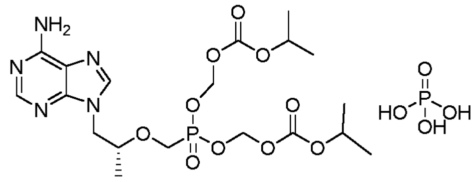

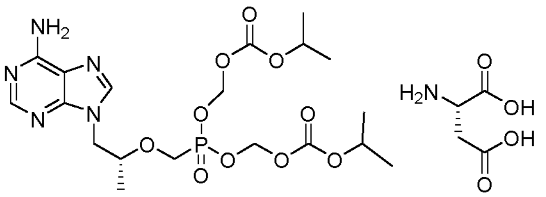

and

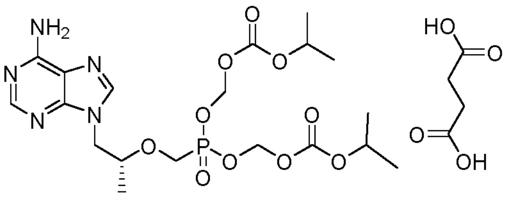

.

In some embodiments of the present application, the pharmaceutical combination described herein comprises a compound of formula **I** or a pharmaceutically acceptable salt thereof, and tenofovir or a pharmaceutically acceptable prodrug thereof or a pharmaceutically acceptable salt thereof. In some embodiments, the pharmaceutical combination described herein comprises a compound of formula **I** or a pharmaceutically acceptable salt thereof, and tenofovir alafenamide or tenofovir disoproxil or a pharmaceutically acceptable salt thereof. In some embodiments, the pharmaceutical combination described herein comprises a compound of formula **I** or a pharmaceutically acceptable salt thereof, and tenofovir disoproxil fumarate or tenofovir alafenamide fumarate.

In some embodiments of the present application, the compound of formula **I** or the pharmaceutically acceptable salt thereof of the pharmaceutical combination may be administered once every day, twice every day, once every two days, once every three days, once every four days, once every five days, once every six days, once every week, once every two weeks, or once every three weeks.

In some embodiments of the present application, the compound of formula **I** or the pharmaceutically acceptable salt thereof of the pharmaceutical combination is administered at a dose of 10 mg or 50 mg (the weight of the compound of formula **I)** each time.

In some embodiments of the present application, the compound of formula **I** or the pharmaceutically acceptable salt thereof of the pharmaceutical combination is administered at 0.0001 to 20 mg/kg weight (the weight of the compound of formula **I).**

In some embodiments of the present application, the compound of formula **I** or the pharmaceutically acceptable salt thereof of the pharmaceutical combination is administered at 1-1500 mg or 10-1000 mg each time.

In some embodiments of the present application, tenofovir or the pharmaceutically acceptable prodrug thereof or the pharmaceutically acceptable salt thereof of the pharmaceutical combination may be administered once every day, twice every day, or once every two days, or once every three days, or once every four days, or once every five days, or once every six days, or once every seven days. In some embodiments of the present application, tenofovir or the pharmaceutically acceptable prodrug thereof or the pharmaceutically acceptable salt thereof of the pharmaceutical combination may be administered once every day.

In some embodiments of the present application, for patients with impaired renal function, tenofovir or the pharmaceutically acceptable prodrug thereof or the pharmaceutically acceptable salt thereof is administered once every 24 hours to patients in which creatinine clearance (mL/min) ≥ 50, once every 48 hours to patients in which creatinine clearance (mL/min) ≥ 30-49, and once every 72-96 hours to a patient in which creatinine clearance (mL/min) ≥ 10-29; for hemodialysis patients, tenofovir or the pharmaceutically acceptable prodrug thereof or the pharmaceutically acceptable salt thereof is administered once every 7 days or after about 12 hours, in total, of hemodialysis.

In some embodiments of the present application, 300 mg of tenofovir disoproxil fumarate is administered each time. In some embodiments of the present application, 25 mg of tenofovir alafenamide or 28 mg of tenofovir alafenamide fumarate is administered each time.

In some embodiments of the present application, an average daily dose of tenofovir or the pharmaceutically

acceptable prodrug thereof or the pharmaceutically acceptable salt thereof of the pharmaceutical combination is 0.05 mg to 500 mg (the weight of tenofovir). In some embodiments of the present application, an average daily dose of tenofovir or the pharmaceutically acceptable prodrug thereof or the pharmaceutically acceptable salt thereof of the pharmaceutical combination is 0.1 mg to 400 mg (the weight of tenofovir). In some embodiments of the present application, an average daily dose of tenofovir or the pharmaceutically acceptable prodrug thereof or the pharmaceutically acceptable salt thereof of the pharmaceutical combination is 1 mg to 300 mg (the weight of tenofovir). In some embodiments of the present application, an average daily dose of tenofovir or the pharmaceutically acceptable prodrug thereof or the pharmaceutically acceptable salt thereof of the pharmaceutical combination is 5 mg to 200 mg (the weight of tenofovir). In some embodiments of the present application, an average daily dose of tenofovir or the pharmaceutically acceptable prodrug thereof or the pharmaceutically acceptable salt thereof of the pharmaceutical combination is 8 mg to 190 mg (the weight of tenofovir). In some embodiments of the present application, an average daily dose of tenofovir or the pharmaceutically acceptable prodrug thereof or the pharmaceutically acceptable salt thereof of the pharmaceutical combination is 10 mg to 185 mg (the weight of tenofovir). In some embodiments of the present application, an average daily dose of tenofovir or the pharmaceutically acceptable prodrug thereof or the pharmaceutically acceptable salt thereof of the pharmaceutical combination is 14 mg to 140 mg (the weight of tenofovir).

In some embodiments of the present application, entecavir or the pharmaceutically acceptable salt thereof or the solvate thereof of the pharmaceutical combination may be administered once every day, twice every day, or once every two days.

In some embodiments of the present application, entecavir or the pharmaceutically acceptable salt thereof or the solvate thereof of the pharmaceutical combination may be administered at a dose of 0.5 mg or 1.0 mg (the weight of entecavir) each time.

In some embodiments of the present application, an average daily dose of entecavir or the pharmaceutically acceptable salt thereof or the solvate thereof of the pharmaceutical combination is 0.005 mg to 10.0 mg. In some embodiments of the present application, an average daily dose of entecavir or the pharmaceutically acceptable salt thereof or the solvate thereof of the pharmaceutical combination is 0.05 mg to 5.0 mg. In some embodiments of the present application, an average daily dose of entecavir or the pharmaceutically acceptable salt thereof or the solvate thereof of the pharmaceutical combination is 0.10 mg to 2.0 mg. In some embodiments of the present application, an average daily dose of entecavir or the pharmaceutically acceptable salt thereof or the solvate thereof of the pharmaceutical combination is 0.25 mg to 2.0 mg. In some embodiments of the present application, an average daily dose of entecavir or the pharmaceutically acceptable salt thereof or the solvate thereof of the pharmaceutical combination is 0.5 mg to 1.0 mg.

In some embodiments of the present application, the compound of formula **I** or the pharmaceutically acceptable salt thereof of the pharmaceutical combination is administered at 1-1500 mg or 10-1000 mg (the weight of the compound of formula **I)** each time, and an average daily dose of tenofovir or the pharmaceutically acceptable prodrug thereof or the pharmaceutically acceptable salt thereof is 0.05 mg to 500 mg (the weight of tenofovir).

In some embodiments of the present application, an average-daily-dose ratio of the compound of formula **I** or the pharmaceutically acceptable salt thereof to tenofovir or the pharmaceutically acceptable prodrug thereof or the pharmaceutically acceptable salt thereof in the pharmaceutical combination (the compound of formula **I**:tenofovir, by weight) is selected from 500:1 to 1:1000. In some embodiments, an average-daily-dose ratio (by weight) of the compound of formula **I** or the pharmaceutically acceptable salt thereof to tenofovir or the pharmaceutically acceptable prodrug thereof or the pharmaceutically acceptable salt thereof is selected from the group consisting of 500:1, 450:1, 400:1, 350:1, 300:1, 250:1, 200:1, 150:1, 100:1, 95:1, 90:1, 85:1, 80:1, 79:1, 78:1, 77:1, 76:1, 75:1, 74:1, 73:1, 72:1, 71:1, 70:1, 69:1, 68:1, 67:1, 66:1, 65:1, 60:1, 55:1, 50:1, 45:1, 40:1, 35:1, 30:1, 25:1, 30:1, 25:1, 20:1, 15:1, 10:1, 5:1, 1:1, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950, and 1:1000, or a range formed by any of the above ratios.

In some embodiments of the present application, an average-daily-dose ratio of the compound of formula **I** or the pharmaceutically acceptable salt thereof to tenofovir or the pharmaceutically acceptable prodrug thereof or the pharmaceutically acceptable salt thereof in the pharmaceutical combination (a molar ratio of free compounds) is selected from 100:1 to 1:100. In some embodiments, an average-daily-dose ratio of the compound of formula **I** or the pharmaceutically acceptable salt thereof to tenofovir or the pharmaceutically acceptable prodrug thereof or the pharmaceutically acceptable salt thereof (a molar ratio of free compounds, the compound of formula **I**:tenofovir) is selected from the group consisting of 100:1, 98:1, 96:1, 94:1, 92:1, 90:1, 88:1, 86:1, 84:1, 82:1, 80:1, 78:1, 76:1, 74:1, 72:1, 70:1, 68:1, 66:1, 64:1, 62:1, 60:1, 58:1, 56:1, 54:1, 52:1, 50:1, 48:1, 46:1, 45:1, 44:1, 43:1, 42:1, 41:1, 40:1, 39:1, 38:1, 37:1, 36:1, 35:1, 34:1, 33:1, 32:1, 31:1, 30:1, 29:1, 28:1, 27:1, 26:1, 25:1, 24:1, 23:1, 22:1, 21:1, 20:1, 18:1, 16:1, 14:1, 12:1, 10:1, 8:1, 6:1, 4:1, 2:1, 1:1, 1:2, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:38, 1:40, 1:42, 1:44, 1:46, 1:48, 1:50, 1:52, 1:54, 1:56, 1:58, 1:60, 1:62, 1:64, 1:66, 1:68, 1:70, 1:72, 1:74, 1:76, 1:78, 1:80, 1:82, 1:84, 1:86, 1:88, 1:90,

1:92, 1:94, 1:96, 1:98, and 1:100, or a range formed by any of the above ratios.

In some embodiments of the present application, an average-daily-dose ratio (by weight) of the compound of formula **I** or the pharmaceutically acceptable salt thereof (the compound of formula **I** calculated as free compound) to tenofovir disoproxil fumarate in the pharmaceutical combination is selected from 1:300 to 1500:300, or 10:300 to 1000:300. In some embodiments, an average-daily-dose ratio (by weight) of the compound of formula **I** or the pharmaceutically acceptable salt thereof (the compound of formula **I** calculated as free compound) to tenofovir disoproxil fumarate in the pharmaceutical combination is selected from the group consisting of 10:300, 20:300, 30:300, 40:300, 50:300, 60:300, 70:300, 80:300, 90:300, 100:300, 110:300, 120:300, 130:300, 140:300, 150:300, 160:300, 170:300, 180:300, 190:300, 200:300, 210:300, 220:300, 230:300, 240:300, 250:300, 260:300, 270:300, 280:300, 290:300, 300:300, 310:300, 320:300, 330:300, 340:300, 350:300, 360:300, 370:300, 380:300, 390:300, 400:300, 410:300, 420:300, 430:300, 440:300, 450:300, 460:300, 470:300, 480:300, 490:300, 500:300, 510:300, 520:300, 530:300, 540:300, 550:300, 560:300, 570:300, 580:300, 590:300, 600:300, 610:300, 620:300, 630:300, 640:300, 650:300, 660:300, 670:300, 680:300, 690:300, 700:300, 710:300, 720:300, 730:300, 740:300, 750:300, 760:300, 770:300, 780:300, 790:300, and 800:300, or a range formed by any of the above ratios.

In some embodiments of the present application, an average-daily-dose ratio (by weight) of the compound of formula **I** or the pharmaceutically acceptable salt thereof (the compound of formula **I** calculated as free compound) to tenofovir alafenamide in the pharmaceutical combination is selected from 1:25 to 1500:25, or 10:25 to 1000:25. In some embodiments, an average-daily-dose ratio (by weight) of the compound of formula **I or** the pharmaceutically acceptable salt thereof (the compound of formula **I** calculated as free compound) to tenofovir alafenamide in the pharmaceutical combination is selected from the group consisting of 10:25, 20:25, 30:25, 40:25, 50:25, 60:25, 70:25, 80:25, 90:25, 100:25, 110:25, 120:25, 130:25, 140:25, 150:25, 160:25, 170:25, 180:25, 190:25, 200:25, 210:25, 220:25, 230:25, 240:25, 250:25, 260:25, 270:25, 280:25, 290:25, 300:25, 310:25, 320:25, 330:25, 340:25, 350:25, 360:25, 370:25, 380:25, 390:25, 400:25, 410:25, 420:25, 430:25, 440:25, 450:25, 460:25, 470:25, 480:25, 490:25, 500:25, 510:25, 520:25, 530:25, 540:25, 550:25, 560:25, 570:25, 580:25, 590:25, 600:25, 610:25, 620:25, 630:25, 640:25, 650:25, 660:25, 670:25, 680:25, 690:25, 700:25, 710:25, 720:25, 730:25, 740:25, 750:25, 760:25, 770:25, 780:25, 790:25, and 800:25, or a range formed by any of the above ratios.

In some embodiments of the present application, an average-daily-dose ratio (by weight) of the compound of formula **I** or the pharmaceutically acceptable salt thereof (the compound of formula **I** calculated as free compound) to tenofovir alafenamide fumarate in the pharmaceutical combination is selected from 1:28 to 1500:28, or 10:28 to 1000:28. In some embodiments, an average-daily-dose ratio (by weight) of the compound of formula **I** or the pharmaceutically acceptable salt thereof (the compound of formula **I** calculated as free compound) to tenofovir alafenamide fumarate in the pharmaceutical combination is selected from the group consisting of 10:28, 20:28, 30:28, 40:28, 50:28, 60:28, 70:28, 80:28, 90:28, 100:28, 110:28, 120:28, 130:28, 140:28, 150:28, 160:28, 170:28, 180:28, 190:28, 200:28, 210:28, 220:28, 230:28, 240:28, 280:28, 260:28, 270:28, 280:28, 290:28, 300:28, 310:28, 320:28, 330:28, 340:28, 350:28, 360:28, 370:28, 380:28, 390:28, 400:28, 410:28, 420:28, 430:28, 440:28, 450:28, 460:28, 470:28, 480:28, 490:28, 500:28, 510:28, 520:28, 530:28, 540:28, 550:28, 560:28, 570:28, 580:28, 590:28, 600:28, 610:28, 620:28, 630:28, 640:28, 650:28, 660:28, 670:28, 680:28, 690:28, 700:28, 710:28, 720:28, 730:28, 740:28, 750:28, 760:28, 770:28, 780:28, 790:28, and 800:28, or a range formed by any of the above ratios.

In some embodiments of the present application, the compound of formula **I** or the pharmaceutically acceptable salt thereof and tenofovir or the pharmaceutically acceptable prodrug thereof or the pharmaceutically acceptable salt thereof in the pharmaceutical combination are each administered in single dose or multiple dose form.

In some embodiments of the present application, the compound of formula **I** or the pharmaceutically acceptable salt thereof of the pharmaceutical combination is administered at 1-1500 mg or 10-1000 mg each time, and an average daily dose of entecavir or the pharmaceutically acceptable salt thereof or the solvate thereof is 0.005 mg to 10.0 mg.

In some embodiments of the present application, an average-daily-dose ratio (by weight) of the compound of formula **I** or the pharmaceutically acceptable salt thereof to entecavir or the pharmaceutically acceptable salt thereof or the solvate thereof is selected from 1000:1 to 1:1000. In some embodiments, an average-daily-dose ratio of the compound of formula **I** or the pharmaceutically acceptable salt thereof to entecavir or the pharmaceutically acceptable salt thereof or the solvate thereof (the compound of formula **I**:entecavir, by weight) is selected from the group consisting of 1000:1, 950:1, 900:1, 850:1, 800:1, 750:1, 700:1, 650:1, 600:1, 550:1, 500:1, 450:1, 400:1, 350:1, 300:1, 250:1, 200:1, 150:1, 100:1, 90:1, 80:1, 70:1, 60:1, 50:1, 40:1, 30:1, 20:1, 10:1, 1:1., 1:10, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950, and 1:1000, or a range formed by any of the above ratios.

In some embodiments of the present application, an average-daily-dose ratio of the compound of formula **I** or the pharmaceutically acceptable salt thereof to entecavir or the pharmaceutically acceptable salt thereof or the solvate thereof in the pharmaceutical combination (a molar ratio of free compounds) is selected from 100:1 to 1:100. In some embodiments, an average-daily-dose ratio of the compound of formula **I** or the pharmaceutically acceptable salt thereof to entecavir or the pharmaceutically acceptable salt thereof or the solvate thereof (a molar ratio of free compounds, the

compound of formula **I**:entecavir) is selected from the group consisting of 100:1, 98:1, 96:1, 94:1, 92:1, 90:1, 88:1, 86:1, 84:1, 82:1, 80:1, 78:1, 76:1, 74:1, 72:1, 70:1, 68:1, 66:1, 64:1, 62:1, 60:1, 58:1, 56:1, 54:1, 52:1, 50:1, 48:1, 46:1, 44:1, 42:1, 40:1, 38:1, 36:1, 34:1, 32:1, 30:1, 28:1, 26:1, 24:1, 22:1, 20:1, 18:1, 16:1, 14:1, 12:1, 10:1, 8:1, 6:1, 4:1, 2:1, 1:1, 1:2, 1:4, 1:6, 1:8, 1:10, 1:12, 1:14, 1:16, 1:18, 1:20, 1:22, 1:24, 1:26, 1:28, 1:30, 1:32, 1:34, 1:36, 1:38, 1:40, 1:42, 1:44, 1:46, 1:48, 1:50, 1:52, 1:54, 1:56, 1:58, 1:60, 1:62, 1:64, 1:66, 1:68, 1:70, 1:72, 1:74, 1:76, 1:78, 1:80, 1:82, 1:84, 1:86, 1:88, 1:90, 1:92, 1:94, 1:96, 1:98, and 1:100, or a range formed by any of the above ratios.

In some embodiments of the present application, an average-daily-dose ratio (by weight) of the compound of formula **I** or the pharmaceutically acceptable salt thereof (the compound of formula **I** calculated as free compound) to entecavir monohydrate in the pharmaceutical combination is selected from 1:0.5 to 1500:0.5, or 10:0.5 to 1000:0.5. In some embodiments, an average-daily-dose ratio (by weight) of the compound of formula **I** or the pharmaceutically acceptable salt thereof (the compound of formula **I** calculated as free compound) to entecavir monohydrate in the pharmaceutical combination is selected from the group consisting of 10:0.5, 20:0.5, 30:0.5, 40:0.5, 50:0.5, 60:0.5, 70:0.5, 80:0.5, 90:0.5, 100:0.5, 110:0.5, 120:0.5, 130:0.5, 140:0.5, 150:0.5, 160:0.5, 170:0.5, 180:0.5, 190:0.5, 200:0.5, 210:0.5, 220:0.5, 230:0.5, 240:0.5, 250:0.5, 260:0.5, 270:0.5, 280:0.5, 290:0.5, 300:0.5, 310:0.5, 320:0.5, 330:0.5, 340:0.5, 350:0.5, 360:0.5, 370:0.5, 380:0.5, 390:0.5, 400:0.5, 410:0.5, 420:0.5, 430:0.5, 440:0.5, 450:0.5, 460:0.5, 470:0.5, 480:0.5, 490:0.5, 500:0.5, 510:0.5, 520:0.5, 530:0.5, 540:0.5, 550:0.5, 560:0.5, 570:0.5, 580:0.5, 590:0.5, 600:0.5, 610:0.5, 620:0.5, 630:0.5, 640:0.5, 650:0.5, 660:0.5, 670:0.5, 680:0.5, 690:0.5, 700:0.5, 710:0.5, 720:0.5, 730:0.5, 740:0.5, 750:0.5, 760:0.5, 770:0.5, 780:0.5, 790:0.5, and 800:0.5, or a range formed by any of the above ratios.

In some embodiments of the present application, the compound of formula **I** or the pharmaceutically acceptable salt thereof and entecavir or the pharmaceutically acceptable salt thereof or the solvate thereof in the pharmaceutical combination are each administered in single dose or multiple dose form.

In some embodiments of the present application, the pharmaceutical combination is a fixed combination. In some embodiments, the fixed combination is in the form of a solid pharmaceutical composition.

In some embodiments, the compound of formula **I** or the pharmaceutically acceptable salt thereof and tenofovir or the pharmaceutically acceptable prodrug thereof or the pharmaceutically acceptable salt thereof in the fixed combination are present in the same solid pharmaceutical composition.

In some embodiments, the compound of formula **I** or the pharmaceutically acceptable salt thereof and entecavir or the pharmaceutically acceptable salt thereof or the solvate thereof in the fixed combination are present in the same solid pharmaceutical composition.

In some embodiments of the present application, the pharmaceutical combination is a non-fixed combination. In some embodiments, the compound of formula **I** or the pharmaceutically acceptable salt thereof and entecavir or the pharmaceutically acceptable salt thereof or the solvate thereof in the non-fixed combination are each in the form of a solid pharmaceutical composition.

In some embodiments, the compound of formula **I** or the pharmaceutically acceptable salt thereof and tenofovir or the pharmaceutically acceptable prodrug thereof or the pharmaceutically acceptable salt thereof in the non-fixed combination are each in the form of a solid pharmaceutical composition, and the solid pharmaceutical composition of the compound of formula **I** or the pharmaceutically acceptable salt thereof and the solid pharmaceutical composition of tenofovir or the pharmaceutically acceptable prodrug thereof or the pharmaceutically acceptable salt thereof are present in the same sachet. In some embodiments, the compound of formula **I** or the pharmaceutically acceptable salt thereof and tenofovir or the pharmaceutically acceptable prodrug thereof or the pharmaceutically acceptable salt thereof in the non-fixed combination are each in the form of a solid pharmaceutical composition, and the solid pharmaceutical composition of the compound of formula **I** or the pharmaceutically acceptable salt thereof and the solid pharmaceutical composition of tenofovir or the pharmaceutically acceptable prodrug thereof or the pharmaceutically acceptable salt thereof are not present in the same sachet.

In some embodiments, the compound of formula **I** or the pharmaceutically acceptable salt thereof and entecavir or the pharmaceutically acceptable salt thereof or the solvate thereof in the non-fixed combination are each in the form of a solid pharmaceutical composition, and the solid pharmaceutical composition of the compound of formula **I** or the pharmaceutically acceptable salt thereof and the solid pharmaceutical composition of entecavir or the pharmaceutically acceptable salt thereof or the solvate thereof are present in the same sachet. In some embodiments, the compound of formula **I** or the pharmaceutically acceptable salt thereof and entecavir or the pharmaceutically acceptable salt thereof or the solvate thereof in the non-fixed combination are each in the form of a solid pharmaceutical composition, and the solid pharmaceutical composition of the compound of formula **I** or the pharmaceutically acceptable salt thereof and the solid pharmaceutical composition of entecavir or the pharmaceutically acceptable salt thereof or the solvate thereof are not present in the same sachet.

In some embodiments of the present application, the solid pharmaceutical composition is selected from the group consisting of a tablet and a capsule.

In another aspect, the present application further provides use of the pharmaceutical combination of the present application for preparing a medicament for treating hepatitis B virus infection. The pharmaceutical combination is as

described above.

In another aspect, the present application further provides a method for treating hepatitis B virus infection comprising administering to an individual in need an effective amount of the pharmaceutical combination of the present application. The pharmaceutical combination is as described above.

In another aspect, the present application further provides the pharmaceutical combination disclosed herein for use in treating hepatitis B virus infection. The pharmaceutical combination is as described above.

In another aspect, the present application further provides use of the pharmaceutical combination disclosed herein for treating hepatitis B virus infection. The pharmaceutical combination is as described above.

In some embodiments of the present application, the present application further provides a kit for use in treating hepatitis B virus infection comprising: (a) a first pharmaceutical composition comprising a compound of formula **I** or a pharmaceutically acceptable salt thereof as an active ingredient; and (b) a second pharmaceutical composition comprising a reverse transcriptase inhibitor or a pharmaceutically acceptable prodrug thereof or a pharmaceutically acceptable salt thereof or a solvate thereof as an active ingredient; and optionally (c) instructions for use of the compound of formula **I** or the pharmaceutically acceptable salt thereof in combination with the reverse transcriptase inhibitor or the pharmaceutically acceptable prodrug thereof or the pharmaceutically acceptable salt thereof or the solvate thereof.

In some embodiments of the present application, the reverse transcriptase inhibitor or the pharmaceutically acceptable prodrug thereof or the pharmaceutically acceptable salt thereof is as described above.

In some embodiments of the present application, the present application provides use of the compound of formula **I** or the pharmaceutically acceptable salt thereof and tenofovir or the pharmaceutically acceptable prodrug thereof or the pharmaceutically acceptable salt thereof for preparing a medicament for treating hepatitis B virus infection, wherein the compound of formula **I** or the pharmaceutically acceptable salt thereof and tenofovir or the pharmaceutically acceptable prodrug thereof or the pharmaceutically acceptable salt thereof are each prepared into a pharmaceutical composition.

In some embodiments of the present application, the present application further provides a kit for use in treating hepatitis B virus infection comprising: (a) a first pharmaceutical composition comprising a compound of formula **I** or a pharmaceutically acceptable salt thereof as an active ingredient; and (b) a second pharmaceutical composition comprising tenofovir or a pharmaceutically acceptable prodrug thereof or a pharmaceutically acceptable salt thereof as an active ingredient; and optionally (c) instructions for use of the compound of formula I or the pharmaceutically acceptable salt thereof in combination with tenofovir or the pharmaceutically acceptable prodrug thereof or the pharmaceutically acceptable salt thereof.

In some embodiments of the present application, the present application provides use of the compound of formula **I** or the pharmaceutically acceptable salt thereof and entecavir or the pharmaceutically acceptable salt thereof or the solvate thereof for preparing a medicament for treating hepatitis B virus infection, wherein the compound of formula **I** or the pharmaceutically acceptable salt thereof and entecavir or the pharmaceutically acceptable salt thereof or the solvate thereof are each prepared into a pharmaceutical combination.

In some embodiments of the present application, the present application further provides a kit for use in treating hepatitis B virus infection comprising: (a) a first pharmaceutical composition comprising a compound of formula **I** or a pharmaceutically acceptable salt thereof as an active ingredient; and (b) a second pharmaceutical composition comprising entecavir or a solvate thereof as an active ingredient; and optionally (c) instructions for use of the compound of formula **I** or the pharmaceutically acceptable salt thereof in combination with entecavir or a pharmaceutically acceptable salt thereof or the solvate thereof.

The compound of formula **I** or the pharmaceutically acceptable salt thereof

The compound of formula **I** is in the prior art; it is chemically named 2-((1r,4r)-4-(2-(6-chloro-7-((3,4,5-trifluorophenyl)carbamoyl)-2,3-dihydro-1H-pyrrolin-5-yl)-2-oxalylamino)-4-m ethylcyclohexyl)acetic acid and has the following structural formula:

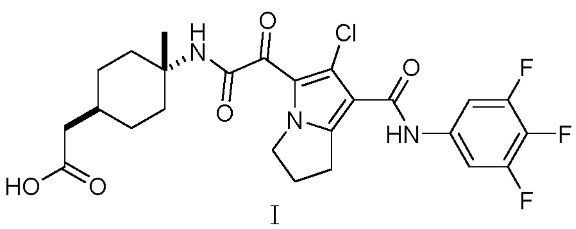

I

For the preparation and chemical properties of the compounds of formula **I** or the pharmaceutically acceptable

salt thereof, reference can be made to WO2019223791.

In some embodiments, the pharmaceutical composition of the compound of formula **I** or the pharmaceutically acceptable salt thereof is selected from a solid pharmaceutical composition, preferably selected from the group consisting of a tablet and a capsule.

In some embodiments, the compound of formula **I** or the pharmaceutically acceptable salt thereof is in the form of a pharmaceutical composition in single doses of 1 mg to 100 mg, preferably a pharmaceutical composition in single doses selected from the group consisting of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, and 100 mg, or a range formed by any of the above values.

Entecavir or the pharmaceutically acceptable salt thereof or the solvate thereof, or the pharmaceutical composition thereof

The entecavir or the pharmaceutically acceptable salt thereof or the solvate thereof described herein include entecavir or the pharmaceutically acceptable salt thereof, the solvate of entecavir or the pharmaceutically acceptable salt thereof, or any combination thereof.

As used herein, entecavir is chemically named 2-amino-9-[(1S,3R,4S)-4-hydroxy-3-hydroxymethyl-2-methylenecyclopentyl]-1,9-dihydro-6H-purin-6-one and has the following structural formula:

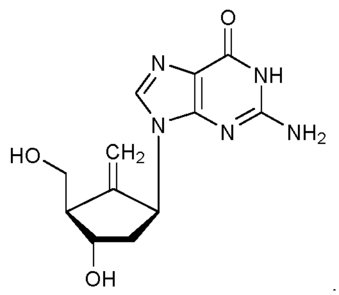

In some embodiments, the pharmaceutically acceptable salt of entecavir is selected from a maleate. In some embodiments, the pharmaceutically acceptable salt of entecavir is selected from a monomaleate.

In some embodiments, the solvate of entecavir is selected from an entecavir hydrate. In some embodiments, the entecavir hydrate is selected from hemihydrate to dihydrate of entecavir. In some embodiments, the entecavir hydrate is selected from entecavir monohydrate.

In some embodiments, entecavir or the pharmaceutically acceptable salt thereof, or the solvate of entecavir or the pharmaceutically acceptable salt thereof, is selected from the group consisting of entecavir monomaleate, entecavir monohydrate, and entecavir monomaleate monohydrate.

In some embodiments, entecavir or the pharmaceutically acceptable salt thereof or the solvate thereof is in the form of a pharmaceutical composition. Preferably, the pharmaceutical composition is selected from a solid pharmaceutical composition. The solid pharmaceutical composition is preferably selected from the group consisting of a tablet and a capsule.

In some embodiments, the pharmaceutical composition of entecavir or the pharmaceutically acceptable salt thereof or the solvate thereof is selected from a pharmaceutical composition in single doses of 0.01 mg to 5 mg, preferably a pharmaceutical composition in single doses selected from the group consisting of 0.01 mg, 0.02 mg, 0.05 mg, 0.08 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, and 5.0 mg, or a range formed by any of the above values.

As used herein, entecavir or the pharmaceutically acceptable salt thereof or the solvate thereof, or the pharmaceutical composition thereof may be selected from a commercially available product (such as entecavir dispersible tablets: Runzhong®).

Tenofovir or the pharmaceutically acceptable prodrug thereof or the pharmaceutically acceptable salt thereof

Tenofovir or the pharmaceutically acceptable prodrug thereof or the pharmaceutically acceptable salt thereof described herein includes a tenofovir free compound or a pharmaceutically acceptable prodrug thereof, or a pharmaceutically acceptable salt of tenofovir or the pharmaceutically acceptable prodrug thereof, or a pharmaceutical composition thereof.

As used herein, tenofovir is chemically named R-9-(2-phosphonylmethoxypropyl)adenine and has the following structural formula:

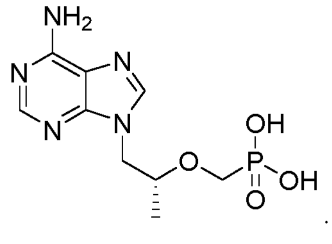

In some embodiments, the pharmaceutically acceptable prodrug of tenofovir is selected from the group consisting of tenofovir alafenamide, tenofovir disoproxil, and tenofovir amibufenamide.

In some embodiments, the pharmaceutically acceptable salt of the tenofovir free compound or the pharmaceutically acceptable prodrug thereof includes a fumarate, an orotate, a disulfonate, a phosphate, a succinate, or an aspartate.

In some embodiments, the pharmaceutically acceptable salt of the tenofovir prodrug is selected from the group consisting of tenofovir disoproxil fumarate, tenofovir alafenamide fumarate, and tenofovir amibufenamide fumarate.

In some embodiments, the pharmaceutically acceptable salt of the tenofovir prodrug is selected from tenofovir alafenamide fumarate. In some embodiments, the ratio of the number of tenofovir alafenamide molecules to the number of fumaric acid molecules in the pharmaceutically acceptable salt of the tenofovir prodrug is 2:1. In some embodiments, the tenofovir alafenamide fumarate is selected from the following structure

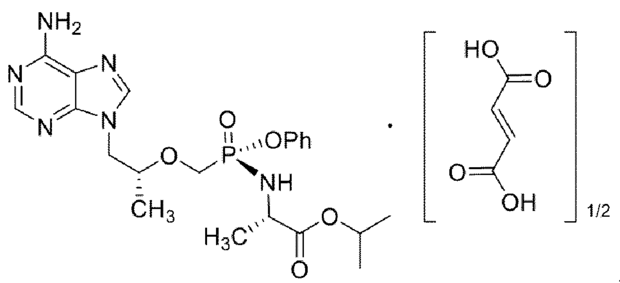

In some embodiments, the pharmaceutically acceptable salt of the tenofovir prodrug is selected from tenofovir disoproxil fumarate. In some embodiments, the ratio of the number of tenofovir disoproxil molecules to the number of fumaric acid molecules in the pharmaceutically acceptable salt of the tenofovir prodrug is 1:1. In some embodiments, the tenofovir disoproxil fumarate is selected from the following structure

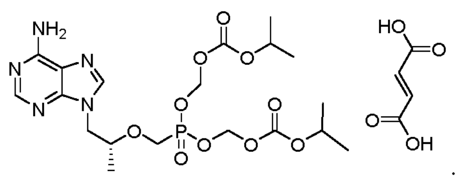

In some embodiments, the pharmaceutically acceptable salt of the tenofovir prodrug is selected from tenofovir amibufenamide fumarate. In some embodiments, the ratio of the number of tenofovir amibufenamide molecules to the number of fumaric acid molecules in the pharmaceutically acceptable salt of the tenofovir prodrug is 1:1. In some embodiments, the tenofovir amibufenamide fumarate is selected from the following structure

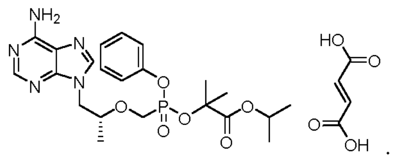

In some embodiments, the pharmaceutically acceptable salt of the tenofovir prodrug is selected from tenofovir disoproxil orotate. In some embodiments, the ratio of the number of tenofovir disoproxil molecules to the number of orotic acid molecules in the pharmaceutically acceptable salt of the tenofovir prodrug is 1:1. In some embodiments, the tenofovir disoproxil orotate is selected from the following structure

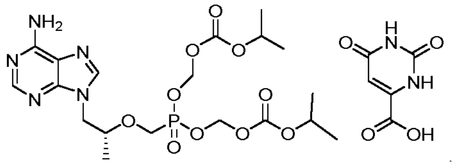

In some embodiments, the pharmaceutically acceptable salt of the tenofovir prodrug is selected from tenofovir disoproxil succinate. In some embodiments, the ratio of the number of tenofovir disoproxil molecules to the number of succinic acid molecules in the pharmaceutically acceptable salt of the tenofovir prodrug is 1:1. In some embodiments, the tenofovir disoproxil succinate is selected from the following structure

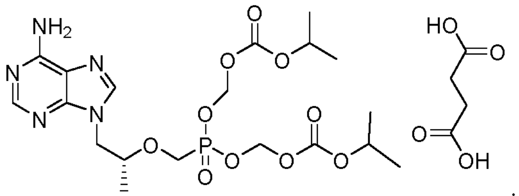

In some embodiments, tenofovir or the pharmaceutically acceptable prodrug thereof or the pharmaceutically acceptable salt thereof is in the form of a pharmaceutical composition. Preferably, the pharmaceutical composition is selected from a solid pharmaceutical composition. The solid pharmaceutical composition is preferably selected from the group consisting of a tablet and a capsule.

In some embodiments, tenofovir or the pharmaceutically acceptable prodrug thereof or the pharmaceutically acceptable salt thereof (calculated based on the tenofovir free compound) is selected from a pharmaceutical composition in single doses of 0.05 mg to 500 mg, preferably a pharmaceutical composition in single doses selected from the group consisting of 0.1 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 200 mg, 250 mg, 300 mg, 350 mg, 400 mg, 450 mg, and 500 mg, or a range formed by any of the above values.

In some embodiments, the pharmaceutical composition of tenofovir disoproxil fumarate is selected from a pharmaceutical composition in single doses of 300 mg.

In some embodiments, the pharmaceutical composition of tenofovir alafenamide is selected from a pharmaceutical composition in single doses of 25 mg.

In some embodiments, the pharmaceutical composition of tenofovir alafenamide fumarate is selected from a pharmaceutical composition in single doses of 28 mg.

As used herein, tenofovir or the pharmaceutically acceptable prodrug thereof or the pharmaceutically acceptable salt thereof may be selected from a commercially available product (such as tenofovir disoproxil fumarate tablets: Qingzhong®, or tenofovir alafenamide fumarate tablets: Vemlidy®).

In some embodiments, the individual is selected from one who has been treated before or one who has not been treated before. In some embodiments, the individual is selected from one who has been treated before with entecavir or a pharmaceutically acceptable salt thereof or a solvate thereof, or tenofovir or a pharmaceutically acceptable prodrug thereof or a pharmaceutically acceptable salt thereof. In some embodiments, the individual is selected from one who has not been treated before. In some embodiments, the individual is selected from one who has been treated before.

In some embodiments, the individual is selected from one who meets the following serum virology criteria: having persistently been serum HBsAg positive for 24 weeks or more or having had evident chronic hepatitis B for 24 weeks, 1000 IU/mL ≤ HbsAg quantitative ≤ 40000 IU/mL.

In some embodiments, the individual is selected from one who has been treated before and meets the following criteria:

1) the subject must be treated with identical doses of the same NA (ETV/TDF) for ≥48 weeks before screening;

2) the medical history shows that HBV DNA inhibition < lower limit of normal 24 weeks before enrollment, and HBV DNA inhibition is defined as <LLOQ during screening;

3) ALT ≤ 5 × ULN.

In some embodiments, the individual is selected from one who has not been treated before and meets the following criteria:

1) must be treatment-naive, namely,

1.1. have never been treated with HBV antiviral drug (NA) or interferon (IFN), or

1.2. have been treated with HBV antiviral drug (NA) or interferon (IFN) but have stopped the medication for 24 weeks or more;

2) HBeAg positive chronic hepatitis B patients, HBV DNA > $10^5$ copies/mL (or > 20,000 IU/mL);

or HBeAg negative patients, HBV DNA > $10^4$ copies/mL (or > 2,000 IU/mL);

3) 1.0 × ULN ≤ ALT ≤ 5 × ULN.

## DEFINITIONS AND DESCRIPTION

Unless otherwise stated, the following terms used herein shall have the following meanings. A certain term, unless otherwise specifically defined, should not be considered uncertain or unclear, but construed according to its common meaning in the field.

As used herein, when the reverse transcriptase inhibitor compound is selected from a solvate form thereof, the amount-of-substance ratio of the compound to the solvent may be selected from the group consisting of 1:0.5, 1:1, 1:1.5, and 1:2, or a range formed by any endpoints, e.g., 1:0.5 to 1:2, 1:0.5 to 1:1.5, or 1:1 to 1:1.5. For example, entecavir is in the form of a non-solvate. For example, the entecavir solvate may be entecavir hydrate. For example, the entecavir hydrate may be entecavir monohydrate.

As used herein, unless otherwise specified, "by weight" means by the weight of the free form of the compound of formula **I,** entecavir or tenofovir.

The term "administer", "administration" or "administering" refers to physically introducing the composition comprising a therapeutic agent to an entity using any of a variety of methods and delivery systems known to those skilled in the art.

The terms "treat", "treating" and "treatment" usually refer to acquiring needed pharmacological effect and/or physiological effect. In terms of partially or fully stabilizing or curing the disease and/or a side effect of the disease, the effect can be therapeutic. As used herein, "treat", "treating" and "treatment" encompass any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound disclosed herein for (i) treating or preventing a specific disease, condition or disorder; (ii) alleviating, relieving or eliminating one or more symptoms of a specific disease, condition or disorder, or (iii) preventing or delaying onset of one or more symptoms of a specific disease, condition or disorder. The amount of the compound of the present application composing the "therapeutically effective amount" varies dependently on the compound, the disease state and its severity, the route of administration, and the age of the mammal to be treated, but can be determined routinely by those skilled in the art in accordance with their knowledge and the present disclosure.

The term "individual" may refer to a mammal. In some embodiments, the subject is a mouse. In some embodiments, the subject is a human.

As used herein, the compound of formula **I** or the pharmaceutically acceptable salt thereof may be administered by any applicable routes and methods, for example, oral administration or parenteral (for example, intravenous) administration. The therapeutically effective amount of the compound of formula **I** or the pharmaceutically acceptable salt thereof includes, but is not limited to, from about 0.0001 to 20 mg/kg/d, for example, from 0.001 to 10 mg/kg/d. The dosing frequency (e.g., once, twice or more times daily) of the compound of formula **I** or the pharmaceutically acceptable salt thereof can be determined according to the requirements of the patient individuals, the severity of the disease, the response of the disease, any treatment-related toxicity, and the age and health of a patient. Administration can be intermittent, for example, in a period of several days, the subject receives a daily dosage of the compound of formula **I** or the pharmaceutically acceptable salt thereof, and in the following period of several days or more days, the patient does not receive the daily dosage of the compound of formula **I** or the pharmaceutically acceptable salt thereof.

Entecavir or the pharmaceutically acceptable salt thereof or the solvate thereof can be administered via various routes including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intraarticular and intrathecal administrations. In some specific embodiments, the drug is administered orally. The amount of entecavir administered can be determined according to the severity of the disease, the response of the disease, any treatment-related toxicity, and the age and health of a patient. For example, entecavir may be administered at a daily dose of 0.005 mg to 10.0 mg. Entecavir can be administered once or more times daily. In some embodiments, entecavir is administered once daily in an oral solid formulation. Tenofovir or the pharmaceutically acceptable prodrug thereof or the pharmaceutically acceptable salt thereof can be administered via various routes including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intraarticular and intrathecal administrations. In some specific embodiments, the drug is administered orally. The amount of tenofovir or the pharmaceutically acceptable prodrug thereof or the pharmaceutically acceptable salt thereof administered can be determined according to the severity of the disease, the response of the disease, any treatment-related toxicity, and the age and health of a patient. For example, tenofovir or the pharmaceutically acceptable prodrug thereof or the pharmaceutically acceptable salt thereof may be administered at a daily dose of 0.05 mg to 500 mg. Tenofovir or the pharmaceutically acceptable prodrug thereof or the pharmaceutically acceptable salt thereof may be administered once or more times daily. In some embodiments, tenofovir or the pharmaceutically acceptable prodrug thereof or the pharmaceutically acceptable salt thereof is administered once daily in an oral solid formulation.

The term "about" shall be understood to include a range of three standard deviations from a mean value or a standard tolerance range in a specific field. In some embodiments, the term "about" shall be understood as a variation not exceeding 0.5. "About" modifies all listed values thereafter. For example, "about 1, 2 and 3" represents "about 1", "about 2" and "about 3".

The term "pharmaceutical combination" refers to combined use of two or more active ingredients in a simultaneous, parallel or sequential manner.

The term "fixed combination" refers to administration of the active ingredients (for example, the compound of formula **I** or entecavir or tenofovir) to a subject simultaneously at a fixed total dose or in a fixed dose proportion, or in the form of a single substance, pharmaceutical composition or formulation. In some embodiments, for example, the active ingredients are present in one tablet, one capsule, or one sachet.

The term "non-fixed combination" refers to simultaneous, parallel or sequential (without specific time limitation) administration of two or more active ingredients as independent substances (for example, a pharmaceutical composition and a pharmaceutical formulation) to an individual, wherein the active ingredients administered to the individual reach therapeutically effective amounts. An example, which can be enumerated, of the non-fixed combination is a cocktail therapy, for example, 2, 3 or more active ingredients are administered. In a non-fixed combination, each active ingredient can be packaged, sold or administered as a fully independent pharmaceutical composition. The "non-fixed combination" further includes combined use of "fixed combinations", or a "fixed combination" and an independent substance of any one or more active ingredients.

The term "pharmaceutical composition" refers to a mixture consisting of one or more of the compounds or the pharmaceutically acceptable salts thereof described herein, or the pharmaceutical combinations thereof disclosed herein and a pharmaceutically acceptable excipient. The pharmaceutical composition is intended to facilitate the administration of the compound or the pharmaceutical combination thereof disclosed herein to a subject.

The term "pharmaceutically acceptable excipients" refers to those which do not have a significant irritating effect on an organic entity and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent and water.

The pharmaceutical composition disclosed herein can be prepared by combining the compound disclosed herein with a suitable pharmaceutically acceptable excipient, and can be formulated, for example, into a solid formulation such as tablet, pill, and capsule.

The pharmaceutical composition disclosed herein can be manufactured using methods well known in the art, such as conventional mixing, dissolving, granulating, dragee-making, levigating, emulsifying, and lyophilizing.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents and the like.

The term "pharmaceutically acceptable salt" refers to salts of the compounds disclosed herein that are within the definition of "pharmaceutically acceptable".

Unless otherwise specified, terms in the singular shall be deemed to include the plural and vice versa. Unless otherwise specified, the word "a" or "an" refers to "at least one". Unless otherwise stated, use of "or" means "and/or".

As used herein, the terms "comprise", "comprises" and "comprising" or equivalents thereof are open-ended statements and mean that elements, components and steps that are not specified may be included in addition to those listed, unless otherwise stated.

The term "single dose" refers to the smallest unit of packaging containing a certain quantity of pharmaceutical product; for example, in a box of seven capsules, each capsule is a single dose; or a vial of injection is a single dose. The term "multiple dose" consists of multiple single doses.

The term "free compound" refers to the presence of the compound of the present application in the free state, particularly to

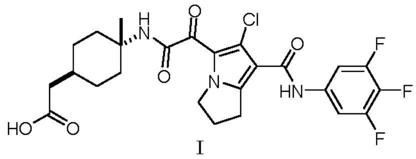

I

,

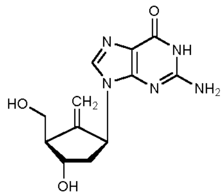

and

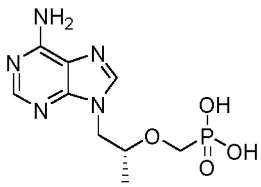

.

The term "solvate" refers to a substance formed by the compound of the present application binding to a pharmaceutically acceptable solvent. The pharmaceutically acceptable solvent includes water, alcohols, etc. The solvate includes both stoichiometric and non-stoichiometric amounts of solvates.

All patents, patent applications and other identified publications are explicitly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

Administration

The content below is not intended to limit the administration of the pharmaceutical combination disclosed herein. The active ingredients in the pharmaceutical combination disclosed herein can be formulated separately, or some or all of the active ingredients are co-formulated. In one embodiment, the pharmaceutical combination disclosed herein can be formulated into a pharmaceutical composition which is suitable for a single dose or multiple doses. The active ingredients in the pharmaceutical combination disclosed herein can be administered separately, or some or all of the active ingredients are co-administered. The ingredients of the pharmaceutical combination disclosed herein can be administered in a substantially asynchronous manner, or some or all of the ingredients are administered in a substantially synchronous manner.

The active ingredients in the pharmaceutical combination disclosed herein can be administered independently, or some or all of the active ingredients are co-administered in various proper routes, including, but not limited to, oral administration or parenteral administration (intravenous, intramuscular, local or subcutaneous routes). In some embodiments, the active ingredients in the pharmaceutical combination disclosed herein can be administered independently, or some or all of the active ingredients are co-administered by means of oral administration or injection, for example,

intravenous injection or intraperitoneal injection.

The active ingredients in the pharmaceutical combination disclosed herein can be in independent suitable dosage forms, or some or all of the active ingredients are co-formulated in a suitable dosage form, including, but not limited to, tablet, lozenge, pill, capsule (for example, hard capsule, soft capsule, enteric capsule and microcapsule), elixir, granule, syrup, injection (intramuscular, intravenous and intraperitoneal), powder, emulsion, suspension, solution, dispersion and dosage forms of slow-released preparations for oral or non-oral administration.

The active ingredients in the pharmaceutical combination disclosed herein can be each independently formulated with a pharmaceutically acceptable carrier and/or excipient, or some or all of the active ingredients are co-formulated with a pharmaceutically acceptable carrier and/or excipient.

The pharmaceutical combination disclosed herein may further comprise an additional therapeutic agent. In one embodiment, the additional therapeutic agent can be a therapeutic agent known in the art for treating hepatitis B virus infection.

In some embodiments, the effective amounts of the compound of formula **I** or the pharmaceutically acceptable salt thereof and entecavir or the pharmaceutically acceptable salt thereof or the solvate thereof of the present application can be administered to an individual in need simultaneously, sequentially or at intervals.

In some embodiments, the effective amounts of the compound of formula **I** or the pharmaceutically acceptable salt thereof and entecavir or the pharmaceutically acceptable salt thereof or the solvate thereof can be administered to an individual in need using the same regimen or different regimens.

In some embodiments, the compound of formula **I** or the pharmaceutically acceptable salt thereof is administered thrice every day, twice every day, once every day, once every two days, once every three days, once every four days, once every five days, once every six days, once every week, once every two weeks or once every three weeks.

In some embodiments, the compound of formula **I** or the pharmaceutically acceptable salt thereof is administered each time at a dose of 1-1500 mg, or 1-1000 mg, or 1-800 mg, or 1-600 mg, or 1-400 mg, or 1-200 mg, or 1-100 mg, preferably 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, 120 mg, 140 mg, 160 mg, 180 mg, 200 mg, 220 mg, 240 mg, 260 mg, 280 mg, 300 mg, 350 mg, 400 mg, 450 mg, 500 mg, 550 mg, 600 mg, 650 mg, 700 mg, 750 mg, or 800 mg, or a range formed by any of the above values.

In some embodiments, the compound of formula **I** or the pharmaceutically acceptable salt thereof is administered once daily at 1-400 mg, or 1-350 mg, or 1-300 mg, or 1-250 mg, or 1-200 mg, or 1-150 mg, or 10-150 mg, or 20-150 mg, or 50-150 mg, or 50-100 mg.

In some embodiments, the compounds of formula **I** or the pharmaceutically acceptable salt thereof is administered once daily at 1-100 mg.

In some embodiments, entecavir or the pharmaceutically acceptable salt thereof or the solvate thereof is administered thrice every day, twice every day, once every day, once every two days, once every three days, once every four days, once every five days, once every six days, once every week, once every two weeks, or once every three weeks.

In some embodiments, entecavir or the pharmaceutically acceptable salt thereof or the solvate thereof is administered each time at a dose of 0.005 mg to 5.0 mg, preferably 0.01 mg, 0.05 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, or 5.0 mg, or a range formed by any of the above values. In some embodiments, entecavir or the pharmaceutically acceptable salt thereof or the solvate thereof is administered each time at a dose of 0.05 mg to 5.0 mg, 0.10 mg to 2.0 mg, 0.25 mg to 2.0 mg, or 0.5 mg to 1.0 mg.

In some embodiments, entecavir or the pharmaceutically acceptable salt thereof or the solvate thereof is administered once daily at a dose of 0.10 mg to 2.0 mg. In some embodiments, entecavir or the pharmaceutically acceptable salt thereof or the solvate thereof may be administered once daily at a dose of 0.5 mg. In some embodiments, the compound of formula **I** is administered once daily at a dose of 1 mg to 100 mg; entecavir or the pharmaceutically acceptable salt thereof or the solvate thereof is administered once daily at a dose of 0.10 mg to 2.0 mg.

In some embodiments, tenofovir or the pharmaceutically acceptable prodrug thereof or the pharmaceutically acceptable salt thereof is administered once every day, twice every day, or once every two days, or once every three days, or once every four days, or once every five days, or once every six days, or once every seven days. In some embodiments of the present application, tenofovir or the pharmaceutically acceptable prodrug thereof or the pharmaceutically acceptable salt thereof of the pharmaceutical combination may be administered once every day. In some

embodiments, tenofovir or the pharmaceutically acceptable prodrug thereof or the pharmaceutically acceptable salt thereof is administered each time at a dose of 0.05 mg to 500 mg, preferably 0.1 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 35 mg, 40 mg, 45 mg, 50 mg, 55 mg, 56 mg, 57 mg, 58 mg, 59 mg, 60 mg, 65 mg, 66 mg, 67 mg, 68 mg, 70 mg, 71 mg, 72 mg, 73 mg, 74 mg, 75 mg, 80 mg, 85 mg, 90 mg, 91 mg, 92 mg, 93 mg, 94 mg, 95 mg, 100 mg, 101 mg, 102 mg, 103 mg, 104 mg, 105 mg, 110 mg, 111 mg, 112 mg, 113 mg, 114 mg, 115 mg, 120 mg, 125 mg, 130 mg, 131 mg, 132 mg, 133 mg, 134 mg, 135 mg, 136 mg, 137 mg, 138 mg, 139 mg, 140 mg, 145 mg, 150 mg, 155 mg, 160 mg, 165 mg, 170 mg, 175 mg, 180 mg, 181 mg, 182 mg, 183 mg, 184 mg, 185 mg, 190 mg, 200 mg, 250 mg, 300 mg, 350 mg, 400 mg, 450 mg, or 500 mg, or a range formed by any of the above values. In some embodiments, tenofovir or the pharmaceutically acceptable prodrug thereof or the pharmaceutically acceptable salt thereof is administered each time at a dose of 0.05 mg to 200 mg, 0.10 mg to 190 mg, 1 mg to 160 mg, or 10 mg to 150 mg.

In some embodiments, tenofovir or the pharmaceutically acceptable prodrug thereof or the pharmaceutically acceptable salt thereof is administered once daily at a dose of 10 mg to 150 mg. In some embodiments, tenofovir or the pharmaceutically acceptable prodrug thereof or the pharmaceutically acceptable salt thereof may be administered once daily at a dose of 15 mg or 135.5 mg.

In some embodiments, tenofovir disoproxil fumarate is administered once daily at 300 mg.

In some embodiments, tenofovir alafenamide is administered once daily at 25 mg.

In some embodiments, tenofovir alafenamide fumarate is administered once daily at 28 mg.

In some embodiments, the compound of formula **I** is administered once daily at a dose of 1 mg to 100 mg; tenofovir or the pharmaceutically acceptable prodrug thereof or the pharmaceutically acceptable salt thereof is administered once daily at a dose of 10 mg to 150 mg.

## TECHNICAL EFFECTS

The pharmaceutical combination of the present application can significantly reduce the HBV DNA level or alleviate other HBV indicators. Moreover, compared to single drugs, the pharmaceutical combination of the present application exhibits enhanced effects, and has good pharmacokinetic properties; the adverse events caused by it are mild, and it can be well tolerated. Compared to single drugs, the pharmaceutical combination can more effectively reduce the levels of HBV markers (including HBsAg, HBsAb, HBeAg, HBeAb, anti-HBc, HBV DNA quantitative, HBV RNA and HbcrAg and the like). These indicate that the pharmaceutical combination disclosed herein has good pharmaceutical value.

## DETAILED DESCRIPTION

For clarity, the present application is further described with the following examples, which are, however, not intended to limit the scope of the present application. All reagents used in the present application are commercially available and can be used without further purification.

### Example 1. HepG2.2.15 Cell Assays

#### 1.1. Materials

##### 1.1.1. Compounds

The compound of formula **I** and entecavir were formulated in 100% DMSO and the stock solutions were stored in a nitrogen cabinet. The concentrations of the compounds are shown in Table 1.

##### 1.1.2. Cell strain

The HepG2.2.15 cell strain was provided by WuXi AppTec.

##### 1.1.3. Reagents

The main reagents used in this study include CellTiter-Glo (Promega, catalog No. G7573), FastStart Universal Probe Mast Mix (Roche, catalog No. 04914058001), and QIAamp 96 DNA Blood Kit (12) (Qiagen, catalog No. 51162).

1.1.4. Instruments

A real-time fluorescence quantitative PCR instrument (Applied Biosystems, model 7900HT Fast Real-Time PCR System) and a multifunctional microplate reader (BioTek, model Synergy2).

1.2. Method

HepG2.2.15 cells were seeded in a 96-well cell culture plate at a density of 60,000 cells/well and then cultured overnight at 37 °C with 5% $CO_2$. The compound of formula **I** and entecavir were formulated in 8 different concentrations (molar ratio 10:1), and meanwhile, single drug controls were set. The compounds were added to a 96-well plate, 3 replicate wells per concentration; the final concentration of DMSO was 1%. The media were replaced with fresh media containing the compounds, and the cells were cultured at 37 °C with 5% $CO_2$ for 3 days. The supernatants in the compound-treated cell plate were collected, and DNA was extracted according to the instructions for QIAamp 96 DNA Blood Kit (12). After the supernatants were collected, CellTiter-Glo was added to measure cell viability.

Table 1. Test concentrations (nM) of compounds

| Compound | Group | | | | | | | |
|---|---|---|---|---|---|---|---|---|
| | 1 | 2 | 3 | 4 | 5 | 6 | 7 | 8 |
| Compound of formula **I** (a) | 15.00 | 11.54 | 8.88 | 6.83 | 5.25 | 4.04 | 3.11 | 2.39 |
| Entecavir (b) | 1.50 | 1.15 | 0.89 | 0.68 | 0.53 | 0.40 | 0.31 | 0.24 |
| Compound of formula **I** in combination with entecavir | a1b1 | a2b2 | a3b3 | a4b4 | a5b5 | a6b6 | a7b7 | a8b8 |
| Note: a1b1 means that the compound of formula **I**-entecavir combination groups use the administration concentration of group 1 of the compound of formula I (a) and the administration concentration of group 1 of entecavir (b), and so on. | | | | | | | | |

HBV DNA was quantified by qPCR. The HBV plasmid DNA was used as a standard and 10-fold diluted from $10^7$ copies/μL to the 7th concentration. A standard curve was fit using the number of HBV DNA copies and the CT value of each standard, and the number of HBV DNA copies in each sample was calculated.

The cytotoxicity of the compounds on HepG2.2.15 cells was measured using a CellTiter-Glo kit. The chemiluminescence intensity (RLU) of each well was measured using a multifunctional microplate reader according to the instructions for the kit.

The data was analyzed using CompuSyn software and the combination index was calculated. The combination indices of 50%, 75%, 90% and 95% inhibition were calculated, and the mean combination index (CIwt) was calculated.

*Mean combination index (CIwt) = [1×CI50+2×CI75+3×CI90+4CI95]/10*

A mean combination index of 0.9-1.1 indicates additive effect; a combination index of <0.9 indicates synergy; a combination index of >1.1 indicates antagonism.

The formulas for calculating the cytotoxicity (cell viability%) and inhibition (%) of the compounds are as follows: Cell viability% = (RLU of compound well - mean RLU of culture medium control wells) / (mean RLU of DMSO control wells - mean RLU of culture medium control wells) × 100%

$$\text{Inhibition\%} = (1 - \text{HBV DNA content of sample} / \text{HBV DNA content of DMSO control}) \times 100$$

1.3. Results

Table 2. Inhibitory activity of *in vitro* combination medication against HBV

| Molar ratio Compound of formula **I**/entecavir | Combination index (CI) | | | | | Mean combination index |
|---|---|---|---|---|---|---|
| | 50% Inhibition | 75% Inhibition | 90% Inhibition | 95% Inhibition | $R^2$ | (CIwt) |
| 10 | 1.37 | 0.95 | 0.78 | 0.72 | 0.9983 | 0.85 |

The mean combination index of the combination of the compound of formula **I** and entecavir against HBV is 0.85, which indicates synergy.

Example 2. Experiments with AAV/HBV Mouse Model

2.1. Materials

2.1.1. Animals

Male C57BL/6 mice, 5 weeks old, free of specific pathogens, purchased from Shanghai Slac Laboratory Animal Co., Ltd., housed in independent ventilated cages. The mice were acclimatized for 3 days and then injected with the AAV/HBV virus.

2.1.2. Solvent and compounds

2.1.2.1. Solvent

Preparation of 10% Solutol: By way of example, the preparation of a 100 mL solution is described: 90 mL of dd.water was precisely drawn off, and 10 mL of solutol in solution form (solid Solutol was placed in a 70-75 °C oven in advance and heated and dissolved for 15-30 min until it was completely dissolved, and the resulting solution was drawn off with a syringe) was added. They were well mixed by stirring to afford a 10% aqueous Solutol solution, prepared once a week. The prepared solution was stored at 2-8 °C.

2.1.2.2. Compounds

The compound of formula **I:** A proper amount of the compound of formula **I** was measured out and dissolved in 10% Solutol (no noticeable particles present). The prepared solution was stored at 2-8 °C.

Entecavir: A proper amount of entecavir was measured out and dissolved in DMSO to prepare a 1 mg/mL solution. The solution was then gradually diluted 1000-fold to 0.001 mg/mL, aliquoted as a stock solution and stored in a -20 °C freezer. One tube of the solution was taken out every day, 10-fold diluted with normal saline to 0.0001 mg/mL, a concentration required by administration, and stored at 2-8 °C for daily administration.

2.1.3. rAAV8-1.3HBV

rAAV8-1.3HBV (type D, ayw) was purchased from FivePlus, Beijing. It was diluted with sterile PBS to $5 \times 10^{11}$ v.g./mL before experiment. Each mouse was injected with 200 μL, i.e., $1 \times 10^{11}$ v.g.

2.1.4. Reagents and instruments

QIAamp96 DNA kit (catalog No. Qiagen-51162), TaqMan® Universal PCR Master Mix (catalog No. Roche-04914058001), hepatitis B virus e antigen detection kit (catalog No. Autobio-CL 0312), and hepatitis B virus surface antigen detection kit (catalog No. Autobio-CL 0310).

A centrifuge (Beckman Allegra X-15R), a microplate reader (BioTek-Synergy 2), a quantitative PCR instrument (ABI-7900), and a spectrophotometer (Thermo-NANODROP 1000).

2.2. Method

2.2.1. Establishment of AAV/HBV mouse model

AAV/HBV injection: rAAV8-1.3HBV was prepared into a 1 × $10^{11}$ v.g./200 μL solution with sterile PBS in advance before injection. Mice were injected with 200 μL of the rAAV8-1.3HBV solution via the tail vein. Blood collection before grouping: On days 14 and 21 post virus injection, about 120 μL of blood was collected from the submandibular vein of the infected mice for serum collection. The whole blood was incubated in a 37 °C incubator for 30 min and then centrifuged at 13,200× g at 4 °C for 3 min, and serum was collected. The serum was stored at -80 °C for HBV DNA, HBeAg and HBsAg assays.

Grouping: On day 27 post virus injection, according to the serum HBV DNA, HBeAg and HBsAg results on days 14 and 21, the mice were randomized into groups of 8 or 4 and correspondingly treated according to the experimental design.

Group number:

i. Solvent group;

ii. Monotherapy group: entecavir;

iii. Monotherapy group: the compound of formula **I** 3 mpk;

iv. Combination group (combo): the compound of formula **I** 3 mpk and entecavir;

v. Monotherapy group: the compound of formula **I** 10 mpk;

vi. Combination group (combo): the compound of formula **I** 10 mpk and entecavir.

2.2.2. *In vivo* experimental design

Compound treatment: On day 28 post infection, all mice were intragastrically administered a solvent or compound. The day of the first administration was counted as day 0 of experiment, and the duration of administration was from day 0 to day 27. Entecavir was administered once daily (QD) and the compound of formula **I** was administered twice daily (BID, 8 hr/16 hr apart). The administration volume was 10 mL/kg.

Table 3. Administration regimen

| Group | Dosage (mg/kg) | Route of administration | Administration interval | Time of administration |
|---|---|---|---|---|
| i. | ---- | i.g. | BID | Days 0-27 |
| ii. | 0.001 mg/kg | | QD | |
| iii. | 3 mg/kg | | BID | |
| iv. | Entecavir 0.001 mg/kg | | QD | |
| | Compound of formula **I** 3 mg/kg | | BID | |
| v. | Compound of formula **I** 10 mg/kg | | BID | |
| vi. | Entecavir 0.001 mg/kg | | QD | |
| | Compound of formula **I** 10 mg/kg | | BID | |
| Note: i.g.: intragastric; QD: once daily. BID: twice daily. The administration of the combination therapy group was consistent with that of the monotherapy groups. | | | | |

Non-endpoint blood collection: On day -1, 3, 7, 14, 21, 28, 35 or 42, about 120 μL of blood was collected from the submandibular vein, and serum was collected and assayed for HBV DNA. If blood collection and administration were on the same day, blood collection was performed about 5 hours after the first administration on that day.

Endpoints of experiment: mice in groups i-iv were euthanized on day 28. Blood was collected from the hearts of the mice, and serum was collected and assayed for HBV DNA. The endpoints of experiment for groups v and vi were on day 42.

2.2.3. Sample analysis

qPCR Quantification of HBV DNA in mouse serum: The DNA was extracted from serum, and the experiment was performed by following the instructions for QIAamp 96 DNA Blood Kit. qPCR conditions: 95 °C, 10 min, 1 cycle; 95 °C, 15 s; 60 °C, 1 min; 40 cycles.

2.2.4. Statistical analysis

Data were expressed as mean ± standard error, and statistical analysis was performed using Student t test.

2.2.5. Results

Table 4. Assay results of HBV DNA in mouse serum 1

| Group number | HBV DNA (log10, copy/μL) time (days) post-dose * | | | | | |
|---|---|---|---|---|---|---|
| | -1 | 3 | 7 | 14 | 21 | 28 |
| i. | 4.38±0.06 | 4.43±0.08 | 4.53±0.07 | 4.59±0.11 | 4.72±0.07 | 4.79±0.08 |
| ii. | 4.19±0.12 | 3.11±0.10 | 2.69±0.11 | 2.75±0.13 | 2.79±0.12 | 2.70±0.17 |
| iii. | 4.46±0.07 | 3.71±0.10 | 3.63±0.09 | 3.71±0.06 | 3.61±0.11 | 3.96±0.11 |
| iv. | 4.38±0.07 | 3.19±0.10 | 2.36±0.10 | 2.20±0.06 | 2.08±0.00 | 2.39±0.11 |

Table 5. Assay results of HBV DNA in mouse serum 2

| Group number | HBV DNA (log10, copy/μL) time (days) post-dose * | | | | | | |
|---|---|---|---|---|---|---|---|
| | -1 | 14 | 21 | 28 | 31 | 35 | 42 |
| v. | 4.45±0.07 | 2.10±0.02 | 2.08±0.00 | 2.12±0.04 | 3.56±0.12 | 4.38±0.09 | 4.64±0.08 |
| vi. | 4.40±0.04 | 2.08±0.00 | 2.08±0.00 | 2.08±0.00 | 2.17±0.05 | 3.15±0.07 | 4.11±0.08 |
| Note: *mean ± standard error. LLOQ was 2.08 log10 copy/μL serum, and values below LLOQ were expressed as 2.08 log10 copy/μL. $p < 0.01$ compared to the solvent group. | | | | | | | |

The above results show that the combination of the compound of formula **I** and entecavir has a significant inhibitory effect on serum HBV DNA compared to the solvent group and is superior to single drugs.

Example 3

1. Test compounds

The compound of formula **I:** prepared into 10 mg capsules and 50 mg capsules.
Entecavir: entecavir tablets, 0.5 mg/tablet (e.g., Runzhong® of Chia Tai Tianqing Pharmaceutical Group Co., Ltd.).
Placebo: placebo of the compound of formula **I.**

2. Enrolled subjects

The subjects should meet the following criteria:

1) Aged between 18 and 70 inclusive, male or female.

2) Serum virology criteria: having persistently been serum HBeAg positive for 6 months or more or having had evident chronic hepatitis B for 6 months, 1000 IU/mL ≤ HBsAg quantitative ≤ 30000 IU/mL.

3) No cirrhosis, as confirmed in the following manner:

Liver tissue biopsies show no cirrhosis (e.g., Metavir score F0-F3, GS score S0-S3) within 1 year before screening or during screening; or

Fiberscan score < 12.4 kPa within 6 months before screening, and abdominal doppler ultrasound exams show no signs of cirrhosis during screening.

The liver biopsy result prevails, if any.

4) The subject must be treatment-naive during screening, namely,

have never been treated with HBV antiviral drug (NA) or interferon (IFN), or

have been treated with HBV antiviral drug (NA) or interferon (IFN) but have stopped the medication for 6 months or more.

5) HBeAg positive chronic hepatitis B patients, HBV DNA > $10^5$ copies/mL (or > 20,000 IU/mL); or HBeAg negative patients, HBV DNA > $10^4$copies/mL (or > 2,000 IU/mL); (HBeAg positive and negative chronic hepatitis B patients were all tested by Roche's Cobas Taqman real-time quantitative PCR, with the lower limit of detection being 20 IU/mL).

6) $1.0 \times \text{ULN} \leq \text{ALT} \leq 5 \times \text{ULN}$.

7) The subject can have good communication with the researchers, understand and comply with the requirements of the study, and understand and sign a consent form.

3. Administration regimen

The capsule/placebo of the compound of formula **I** was administered once daily on an empty stomach (at least 2 hours before or after meals), and the administration should be performed every day at approximately the same time.

Entecavir was administered once daily on an empty stomach (at least 2 hours before or after meals), and the administration should be performed every day at approximately the same time.

4. Effectiveness indicators

The indicators include HBV markers (HBsAg, HBsAb, HBeAg, HBeAb, anti-HBc, HBV DNA quantitative, HBV RNA, and HBcrAg).

Example 4

1. Test compounds

The compound of formula **I:** prepared into 10 mg capsules and 50 mg capsules.

Entecavir: entecavir tablets, 0.5 mg/tablet (e.g., Runzhong® of Chia Tai Tianqing Pharmaceutical Group Co., Ltd.).

Tenofovir: tenofovir disoproxil fumarate tablets, 300 mg/tablet (e.g., Qingzhong® of Chia Tai Tianqing Pharmaceutical Group Co., Ltd.).

Tenofovir: tenofovir alafenamide fumarate tablets, 25 mg/tablet.

Placebo: placebo of the compound of formula **I.**

2. Enrolled subjects

The subjects should meet the following criteria:

1) Aged between 18 and 70 (inclusive), male or female.

2) Serum virology criteria: having persistently been serum HBsAg positive for 24 weeks or more or having had evident chronic hepatitis B for 24 weeks. 1000 IU/mL ≤ HbsAg quantitative ≤ 40000 IU/mL.

3) Having no cirrhosis as judged by the researchers.

4) The subject can have good communication with the researchers, understand and comply with the requirements of the study, and understand and sign a consent form.

The treated patients should meet the following criteria:

5) The subject must be treated with identical doses of the same NA (ETV/TDF) for ≥48 weeks before screening.

6) The medical history shows that HBV DNA inhibition < lower limit of normal 24 weeks before enrollment, and HBV DNA inhibition is defined as <LLOQ during screening.

7) ALT ≤ 5 × ULN.

The treatment-naive patients should meet the following criteria:

8) The subject must be treatment-naive during screening, namely,

♦ have never been treated with HBV antiviral drug (NA) or interferon (IFN), or

♦ have been treated with HBV antiviral drug (NA) or interferon (IFN) but have stopped the medication for 24 weeks or more.

9) HBeAg positive chronic hepatitis B patients, HBV DNA > $10^5$ copies/mL (or > 20,000 IU/mL); or HBeAg negative patients, HBV DNA > $10^4$ copies/mL (or > 2,000 IU/mL).

10) 1.0 × ULN ≤ ALT ≤ 5 × ULN.

3. Administration regimen

The capsule/placebo of the compound of formula **I** was administered once daily on an empty stomach (at least 2 hours before or after meals), and the administration should be performed every day at approximately the same time.
Entecavir was administered once daily on an empty stomach (at least 2 hours before or after meals), and the administration should be performed every day at approximately the same time.
Tenofovir was administered once daily on an empty stomach (at least 2 hours before or after meals), and the administration should be performed every day at approximately the same time.

4. Effectiveness indicators

The indicators include HBV markers (HBsAg, HBsAb, HBeAg, HBeAb, anti-HBc, HBV DNA quantitative, HBV RNA, and HbcrAg).

## Claims

1. A pharmaceutical combination comprising a compound of formula **I** or a pharmaceutically acceptable salt thereof, and a reverse transcriptase inhibitor or a pharmaceutically acceptable prodrug thereof or a pharmaceutically acceptable salt thererof or a solvate thereof,

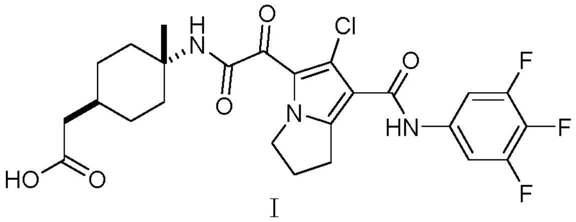

I

.

2. The pharmaceutical combination according to claim 1, wherein the reverse transcriptase inhibitor is selected from the group consisting of entecavir or a pharmaceutically acceptable salt thereof or a solvate thereof, and tenofovir or a pharmaceutically acceptable prodrug thereof or a pharmaceutically acceptable salt thereof.

3. The pharmaceutical combination according to claim 2, wherein entecavir or the pharmaceutically acceptable salt thereof or the solvate thereof is selected from the group consisting of entecavir maleate, entecavir monomaleate, entecavir hydrate, hemihydrate to dihydrate of entecavir, and entecavir monohydrate; optionally, entecavir or the pharmaceutically acceptable salt thereof or the solvate thereof is selected from entecavir monomaleate monohydrate.

4. The pharmaceutical combination according to claim 2 or 3, wherein an average-daily-dose ratio of the compound of formula **I** or the pharmaceutically acceptable salt thereof to entecavir or the pharmaceutically acceptable salt thereof or the solvate thereof is selected from 1000:1 to 1:1000; optionally, the average-daily-dose ratio of the compound of formula **I** or the pharmaceutically acceptable salt thereof to entecavir or the pharmaceutically acceptable salt thereof or the solvate thereof is selected from the group consisting of 1000:1, 950:1, 900:1, 850:1, 800:1, 750:1, 700:1, 650:1, 600:1, 550:1, 500:1, 450:1, 400:1, 350:1, 300:1, 250:1, 200:1, 150:1, 100:1, 90:1, 80:1, 70:1, 60:1, 50:1, 40:1, 30:1, 20:1, 10:1, 1:1., 1:10, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950, and 1:1000, or a range formed by any of the above ratios.

5. The pharmaceutical combination according to any one of claims 2-4, wherein entecavir or the pharmaceutically acceptable salt thereof or the solvate thereof is administered thrice every day, twice every day, once every day, once every two days, once every three days, once every four days, once every five days, once every six days, once every week, once every two weeks, or once every three weeks.

6. The pharmaceutical combination according to any one of claims 2-4, wherein entecavir or the pharmaceutically acceptable salt thereof or the solvate thereof is administered each time at a dose of 0.005 mg to 5.0 mg; optionally, entecavir or the pharmaceutically acceptable salt thereof or the solvate thereof is administered each time at a dose of 0.01 mg, 0.05 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, or 5.0 mg, or a range formed by any of the above values.

7. The pharmaceutical combination according to claim 2, wherein the pharmaceutically acceptable prodrug of tenofovir includes tenofovir disoproxil, tenofovir alafenamide, or tenofovir amibufenamide;

optionally, the pharmaceutically acceptable salt of tenofovir or the pharmaceutically acceptable prodrug thereof is selected from the group consisting of a fumarate, an orotate, a disulfonate, a phosphate, a succinate, and an aspartate;

optionally, tenofovir or the pharmaceutically acceptable prodrug thereof or the pharmaceutically acceptable salt thereof is selected from the group consisting of tenofovir, tenofovir alafenamide fumarate, tenofovir disoproxil fumarate, tenofovir disoproxil orotate, tenofovir disoproxil hemidisulfonate, tenofovir disoproxil phosphate, tenofovir disoproxil succinate, tenofovir disoproxil aspartate, and tenofovir amibufenamide fumarate.

8. The pharmaceutical combination according to claim 2 or 7, wherein an average-daily-dose ratio of the compound of formula **I** or the pharmaceutically acceptable salt thereof to tenofovir or the pharmaceutically acceptable prodrug thereof or the pharmaceutically acceptable salt thereof is selected from 500:1 to 1:1000; optionally, the average-daily-dose ratio of the compound of formula **I** or the pharmaceutically acceptable salt thereof to tenofovir or the pharmaceutically acceptable prodrug thereof or the pharmaceutically acceptable salt thereof is selected from the group consisting of 500:1, 450:1, 400:1, 350:1, 300:1, 250:1, 200:1, 150:1, 100:1, 95:1, 90:1, 85:1, 80:1, 79:1, 78:1, 77:1, 76:1, 75:1, 74:1, 73:1, 72:1, 71:1, 70:1, 69:1, 68:1, 67:1, 66:1, 65:1, 60:1, 55:1, 50:1, 45:1, 40:1, 35:1, 30:1, 25:1, 30:1, 25:1, 20:1, 15:1, 10:1, 5:1, 1:1, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950, and 1:1000, or a range formed by any of the above ratios.

9. The pharmaceutical combination according to any one of claims 2, 7 and 8, wherein tenofovir or the pharmaceutically acceptable prodrug thereof or the pharmaceutically acceptable salt thereof is administered once every day, or twice

every day, or once every two days, or once every three days, or once every four days, or once every five days, or once every six days, or once every seven days.

**10.** The pharmaceutical combination according to any one of claims 2 and 7-9, wherein an average daily dose of tenofovir or the pharmaceutically acceptable prodrug thereof or the pharmaceutically acceptable salt thereof is 0.05 mg to 500 mg; or the average daily dose is 0.1 mg to 400 mg; or the average daily dose is 1 mg to 300 mg; or the average daily dose is 5 mg to 200 mg; or the average daily dose is 8 mg to 190 mg; or the average daily dose is 10 mg to 185 mg; or the average daily dose is 14 mg to 140 mg;

optionally, 300 mg of tenofovir disoproxil fumarate is administered each time;
or 25 mg of tenofovir alafenamide is administered each time;
or 28 mg of tenofovir alafenamide fumarate is administered each time.

**11.** The pharmaceutical combination according to any one of claims 1-10, wherein the compound of formula I or the pharmaceutically acceptable salt thereof is administered once every day, twice every day, once every two days, once every three days, once every four days, once every five days, once every six days, once every week, once every two weeks, or once every three weeks.

**12.** The pharmaceutical combination according to any one of claims 1-11, wherein the compound of formula I or the pharmaceutically acceptable salt thereof is administered each time at a dose of 1 mg to 1500 mg.

**13.** The pharmaceutical combination according to any one of claims 1-12, wherein the pharmaceutical combination is a fixed combination; optionally, the fixed combination is in the form of a solid pharmaceutical composition; optionally, the compound of formula **I** or the pharmaceutically acceptable salt thereof and entecavir or the pharmaceutically acceptable salt thereof or the solvate thereof, or tenofovir or the pharmaceutically acceptable prodrug thereof or the pharmaceutically acceptable salt thereof, in the fixed combination are present in the same solid pharmaceutical composition.

**14.** The pharmaceutical combination according to any one of claims 1-12, wherein the pharmaceutical combination is a non-fixed combination;

optionally, the compound of formula **I** or the pharmaceutically acceptable salt thereof and entecavir or the pharmaceutically acceptable salt thereof or the solvate thereof, or tenofovir or the pharmaceutically acceptable prodrug thereof or the pharmaceutically acceptable salt thereof, in the non-fixed combination are each in the form of a solid pharmaceutical composition;
optionally, the compound of formula **I** or the pharmaceutically acceptable salt thereof and entecavir or the pharmaceutically acceptable salt thereof or the solvate thereof, or tenofovir or the pharmaceutically acceptable prodrug thereof or the pharmaceutically acceptable salt thereof, in the non-fixed combination are each in the form of a solid pharmaceutical composition, and the solid pharmaceutical composition of the compound of formula **I** or the pharmaceutically acceptable salt thereof and the solid pharmaceutical composition of entecavir or the pharmaceutically acceptable salt thereof or the solvate thereof, or tenofovir or the pharmaceutically acceptable prodrug thereof or the pharmaceutically acceptable salt thereof, are present in the same sachet;
optionally, the compound of formula **I** or the pharmaceutically acceptable salt thereof and entecavir or the pharmaceutically acceptable salt thereof or the solvate thereof, or tenofovir or the pharmaceutically acceptable prodrug thereof or the pharmaceutically acceptable salt thereof, in the non-fixed combination are each in the form of a solid pharmaceutical composition, and the solid pharmaceutical composition of the compound of formula **I** or the pharmaceutically acceptable salt thereof and the solid pharmaceutical composition of entecavir or the pharmaceutically acceptable salt thereof or the solvate thereof, or tenofovir or the pharmaceutically acceptable prodrug thereof or the pharmaceutically acceptable salt thereof, are not present in the same sachet.

**15.** Use of the pharmaceutical combination according to any one of claims 1-14 for preparing a medicament for treating hepatitis B virus infection.

**16.** A method for treating hepatitis B virus infection comprising administering to a subject an effective amount of the pharmaceutical combination according to any one of claims 1-14.

**17.** A pharmaceutical combination for use in treating hepatitis B virus infection, wherein the pharmaceutical combination is selected from the pharmaceutical combination according to any one of claims 1-14.

**18.** Use of the pharmaceutical combination according to any one of claims 1-14 for treating hepatitis B virus infection.

**19.** A kit for use in treating hepatitis B virus infection comprising: (a) a first pharmaceutical composition comprising a compound of formula I or a pharmaceutically acceptable salt thereof as an active ingredient; and (b) a second pharmaceutical composition comprising a reverse transcriptase inhibitor or a pharmaceutically acceptable prodrug thereof or a pharmaceutically acceptable salt thereof or a solvate thereof as an active ingredient; and optionally (c) instructions for use of the compound of formula I or the pharmaceutically acceptable salt thereof in combination with the reverse transcriptase inhibitor or the pharmaceutically acceptable prodrug thereof or the pharmaceutically acceptable salt thereof or the solvate thereof,

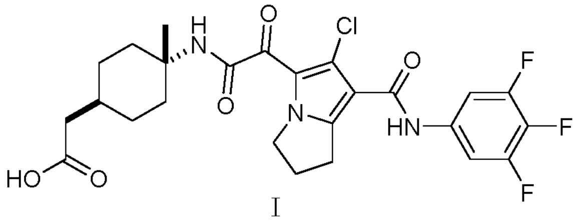

I

**20.** The kit according to claim 19, wherein the reverse transcriptase inhibitor or the pharmaceutically acceptable prodrug thereof or the pharmaceutically acceptable salt thereof or the solvate thereof is selected from the group consisting of entecavir or a pharmaceutically acceptable salt thereof or a solvate thereof, and tenofovir or a pharmaceutically acceptable prodrug thereof or a pharmaceutically acceptable salt thereof.

## INTERNATIONAL SEARCH REPORT

International application No.
**PCT/CN2021/128892**

**A. CLASSIFICATION OF SUBJECT MATTER**

A61K 31/407(2006.01)i； A61K 31/437(2006.01)i； A61K 31/522(2006.01)i； A61K 31/7072(2006.01)i； A61K 45/06(2006.01)i； A61P 31/20(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNTXT; WPABS; DWPI; CNKI; 万方; Web of Science: 正大天晴, 徐中南, 霍丹丹, 贺海鹰, 张弘, 于雅楠, 夏建华, 吡咯嗪, 衣壳蛋白, 核蛋白, 核心蛋白, 抑制剂, 组合, 核苷, 逆转录酶抑制剂, 恩替卡韦, 替诺福韦, CHIA TAI TIANQING, pyrrolizine, nucleoprotein, core protein, capsid, CpAM, combination, tenofovir, entecavir

**C. DOCUMENTS CONSIDERED TO BE RELEVANT**

| Category* | Citation of document, with indication, where appropriate, of the relevant passages | Relevant to claim No. |
|---|---|---|
| Y | WO 2019223791 A1 (CHIA TAI TIANQING PHARMACEUTICAL GROUP CO., LTD. et al.) 28 November 2019 (2019-11-28)<br>abstract, and description, embodiment 25 | 1-20 |
| Y | CN 109790168 A (GILEAD SCIENCES, INC.) 21 May 2019 (2019-05-21)<br>claims 12, 69-70, 72 | 1-20 |
| Y | CN 111788204 A (GILEAD SCIENCES, INC.) 16 October 2020 (2020-10-16)<br>claims 7, 63-64, 66 | 1-20 |
| Y | CN 109640980 A (NOVIRA THERAPEUTICS, INC. et al.) 16 April 2019 (2019-04-16)<br>claims 1 and 6-9 | 1-20 |
| Y | CN 109689059 A (F. HOFFMANN-LA ROCHE AG.) 26 April 2019 (2019-04-26)<br>claims 1 and 6-8 | 1-20 |
| Y | 杨璐 等. (YANG, Lu et al.). "乙型肝炎病毒衣壳蛋白装配调节剂研究进展. (Advances in HBV Capsid Protein Assembly Modulators.)"<br>*中国药理学通报 (Chinese Pharmacological Bulletin),*<br>Vol. 35, No. 11, 28 October 2019 (2019-10-28),<br>pp. 1481-1487 | 1-20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 January 2022** | **29 January 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

INTERNATIONAL SEARCH REPORT

International application No.
PCT/CN2021/128892

C. DOCUMENTS CONSIDERED TO BE RELEVANT

| Category* | Citation of document, with indication, where appropriate, of the relevant passages | Relevant to claim No. |
|---|---|---|
| Y | KO, Chunkyu et al. . "A New Role for Capsid Assembly Modulators To Target Mature Hepatitis B Virus Capsids and Prevent Virus Infection." *Antimicrobial Agents and Chemotherapy.*, Vol. 64, No. 1, 20 December 2019 (2019-12-20), pp. 1-13 | 1-20 |
| A | NIJAMPATNAM, Bhavitavya et al. . "Recent advances in the development of HBV capsid assembly modulators." *Current Opinion in Chemical Biology.*, Vol. 50, 31 December 2019 (2019-12-31), pp. 73-79 | 1-20 |

Form PCT/ISA/210 (second sheet) (January 2015)

**INTERNATIONAL SEARCH REPORT**

International application No.
**PCT/CN2021/128892**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16,18**
because they relate to subject matter not required to be searched by this Authority, namely:

[1] Claim 16 sets forth a method for treating hepatitis B virus infection. Claim 18 sets forth a use of a pharmaceutical composition for treating hepatitis B virus infection. Both claims relate to methods for prevention and treatment of human or animal diseases, and belong to a subject matter that does not require a search as defined in PCT Rule 39.1(iv). The search is performed on the basis of the subject matter of claim 16 or 18 being a use of a pharmaceutical composition in preparing a drug for treating hepatitis B virus infection.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/128892**

| Patent document cited in search report | | | Publication date (day/month/year) | Patent family member(s) | | | Publication date (day/month/year) |
|---|---|---|---|---|---|---|---|
| WO | 2019223791 | A1 | 28 November 2019 | PH | 12020552017 | A1 | 28 June 2021 |
| | | | | CN | 111247151 | A | 05 June 2020 |
| | | | | JP | 2021525267 | A | 24 September 2021 |
| | | | | CA | 3101373 | A1 | 28 November 2019 |
| | | | | SG | 11202011685 Q | A | 30 December 2020 |
| | | | | KR | 20210016402 | A | 15 February 2021 |
| | | | | AU | 2019272481 | A1 | 17 December 2020 |
| | | | | EP | 3805223 | A1 | 14 April 2021 |
| | | | | EP | 3805223 | A4 | 22 December 2021 |
| CN | 109790168 | A | 21 May 2019 | JP | 2019530652 | A | 24 October 2019 |
| | | | | JP | 6774562 | B2 | 28 October 2020 |
| | | | | BR | 112019003415 | A2 | 21 May 2019 |
| | | | | AU | 2017315863 | A1 | 07 March 2019 |
| | | | | AU | 2017315863 | B2 | 05 March 2020 |
| | | | | AU | 2017315863 | C1 | 19 August 2021 |
| | | | | IL | 264800 | A | 28 February 2021 |
| | | | | KR | 20210027520 | A | 10 March 2021 |
| | | | | JP | 2021006568 | A | 21 January 2021 |
| | | | | DO | P2021000112 | A | 15 July 2021 |
| | | | | CL | 2019000473 | A1 | 12 July 2019 |
| | | | | PL | 3504212 | T3 | 22 November 2021 |
| | | | | EC | SP19012917 | A | 29 March 2019 |
| | | | | UY | 37374 | A | 23 March 2018 |
| | | | | EP | 3922634 | A1 | 15 December 2021 |
| | | | | EP | 3922634 | A4 | 15 December 2021 |
| | | | | IL | 280747 | D0 | 29 April 2021 |
| | | | | IL | 280747 | A | 30 September 2021 |
| | | | | SI | 3504212 | T1 | 31 August 2021 |
| | | | | AU | 2020203499 | A1 | 18 June 2020 |
| | | | | AU | 2020203499 | B2 | 21 October 2021 |
| | | | | CO | 2019001634 | A2 | 08 March 2019 |
| | | | | JO | P20190024 | A1 | 16 June 2017 |
| | | | | WO | 2018039531 | A1 | 01 March 2018 |
| | | | | CA | 3033681 | A1 | 01 March 2018 |
| | | | | CA | 3033681 | C | 04 May 2021 |
| | | | | ES | 2879674 | T3 | 22 November 2021 |
| | | | | PH | 12019500393 | A1 | 20 January 2020 |
| | | | | EP | 3504212 | A1 | 03 July 2019 |
| | | | | EP | 3504212 | B1 | 19 May 2021 |
| | | | | KR | 20190039798 | A | 15 April 2019 |
| | | | | KR | 102224081 | B1 | 10 March 2021 |
| | | | | US | 2021251957 | A1 | 19 August 2021 |
| | | | | TW | 201819384 | A | 01 June 2018 |
| | | | | PT | 3504212 | T | 28 July 2021 |
| | | | | CR | 20190100 | A | 02 May 2019 |
| | | | | DO | P2019000040 | A | 15 March 2019 |
| | | | | NZ | 750757 | A | 28 August 2020 |
| | | | | MX | 2019002049 | A | 08 July 2019 |
| | | | | US | 2018161307 | A1 | 14 June 2018 |
| | | | | US | 10328053 | B2 | 25 June 2019 |

Form PCT/ISA/210 (patent family annex) (January 2015)

**INTERNATIONAL SEARCH REPORT**
**Information on patent family members**

International application No.
**PCT/CN2021/128892**

| Patent document cited in search report | Publication date (day/month/year) | Patent family member(s) | Publication date (day/month/year) |
|---|---|---|---|
| | | SG 11201901131V A | 28 March 2019 |
| | | AU 2021240103 A1 | 28 October 2021 |
| | | US 2020061024 A1 | 27 February 2020 |
| | | US 10874640 B2 | 29 December 2020 |
| | | PE 20190631 A1 | 26 April 2019 |
| | | CL 2019001976 A1 | 06 December 2019 |
| CN 111788204 A | 16 October 2020 | US 2021147429 A1 | 20 May 2021 |
| | | CA 3091142 A1 | 29 August 2019 |
| | | WO 2019165374 A1 | 29 August 2019 |
| | | TW 201945366 A | 01 December 2019 |
| | | TW I731309 B | 21 June 2021 |
| | | KR 20200124716 A | 03 November 2020 |
| | | US 2019292187 A1 | 26 September 2019 |
| | | US 10836769 B2 | 17 November 2020 |
| | | AU 2019223182 A1 | 27 August 2020 |
| | | AU 2019223182 B2 | 19 August 2021 |
| | | EP 3759109 A1 | 06 January 2021 |
| | | JP 2021514966 A | 17 June 2021 |
| CN 109640980 A | 16 April 2019 | JP 2019511542 A | 25 April 2019 |
| | | BR 112018071048 A2 | 07 May 2019 |
| | | SG 11201808949 S A | 29 November 2018 |
| | | US 2019365767 A1 | 05 December 2019 |
| | | US 11129834 B2 | 28 September 2021 |
| | | US 2017340642 A1 | 30 November 2017 |
| | | US 10441589 B2 | 15 October 2019 |
| | | EP 3848026 A1 | 14 July 2021 |
| | | SG 10202011827 Y A | 28 January 2021 |
| | | AU 2017248828 A1 | 01 November 2018 |
| | | MX 2018012557 A | 04 July 2019 |
| | | EP 3442524 A2 | 20 February 2019 |
| | | CA 3021068 A1 | 19 October 2017 |
| | | WO 2017181141 A2 | 19 October 2017 |
| | | WO 2017181141 A3 | 11 January 2018 |
| | | IL 262378 D0 | 29 November 2018 |
| | | MA 44671 A | 20 February 2019 |
| | | KR 20180129943 A | 05 December 2018 |
| CN 109689059 A | 26 April 2019 | EP 3503894 A1 | 03 July 2019 |
| | | JP 2019526562 A | 19 September 2019 |
| | | US 2019275052 A1 | 12 September 2019 |
| | | WO 2018036941 A1 | 01 March 2018 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019223791 A